# EUROPÄISCHE PATENTANMELDUNG

(11) **EP 4 838 441 A1**
(43) Veröffentlichungstag der Anmeldung: **11.11.2026**
(21) Anmeldenummer: 26173220.0
(22) Anmeldetag: 20.04.2026
(51) Int. Cl.: G06Q 10/20, G01N 33/00

(54) **AUSGABEAUTOMAT FÜR GASMESSGERÄTE SOWIE VERFAHREN ZUM AUSGEBEN UND VERFAHREN ZUM RÜCKGEBEN EINES GASMESSGERÄTS**

(30) Priorität: 28.04.2025 DE 102025116265; 08.05.2025 DE 102025117788; 30.10.2025 DE 102025144485
(71) Anmelder: Dräger Safety AG & Co. KGaA, 23560 Lübeck (DE)
(72) Erfinder: Brickwedde, Marc, 23558 Lübeck (DE); Hammer, Thielo, 23558 Lübeck (DE)

(57) **Zusammenfassung**

Der erfindungsgemäße Ausgabeautomat (100) vermag mindestens ein Gasmessgerät (1.1, 1.2, 1.3) auszugeben. Das Gasmessgerät (1.1, 1.2, 1.3) ist in einer Aufnahmeeinheit (10.1, 10.2, 10.3) des Ausgabeautomaten (100) eingesetzt. Eine Fluidführungs-Anordnung (30, 11.1, 11.2, 11.3) verbindet einen Gaseinlass mit der Aufnahmeeinheit. In einer verschließenden Position verschließt ein Verschluss (13.1, 13.2, 13.3) die Aufnahmeeinheit, in einer geöffneten Position gibt der Verschluss die Aufnahmeeinheit (10.1, 10.2, 10.3) frei und ermöglicht eine Entnahme. In einem arretierenden Zustand arretiert eine Arretiereinheit (14.1, 14.2, 14.3) den Verschluss (13.1, 13.2, 13.3), in einem freigebenden Zustand gibt die Arretiereinheit (14.1, 14.2, 14.3) den Verschluss frei. Als Reaktion auf die Erfassung einer Benutzereingabe wird geprüft, ob in die Aufnahmeeinheit ein verwendbares Gasmessgerät (1.1, 1.2, 1.3) eingesetzt ist, nämlich ein Gasmessgerät, welches eine Zielgas-Konzentration ausreichend genau zu messen vermag. Falls ein verwendbares Gasmessgerät (1.1, 1.2, 1.3) eingesetzt ist, wird der Verschluss (13.1, 13.2, 13.3) dearretiert.

## Beschreibung

Die Erfindung betrifft einen Ausgabeautomaten für Gasmessgeräte. Weiterhin betrifft die Erfindung ein Ausgabe-Verfahren, um unter Verwendung eines solchen Ausgabeautomaten ein Gasmessgerät auszugeben, sowie ein Rückgabe-Verfahren, um ein Gasmessgerät in einen solchen Ausgabeautomaten zurückzugeben.

Bekanntlich vermag ein Gasmessgerät die Konzentration eines Zielgases in einer Gasprobe zu messen. Ein Sonderfall dieser Messung ist, dass das Gasmessgerät automatisch zu entscheiden vermag, ob die Zielgas-Konzentration in einem vorgegebenen Wertebereich liegt oder nicht. Das Zielgas ist brennbar oder giftig oder auf eine andere Weise für Menschen schädlich oder auch für Menschen lebensnotwendig, es ist beispielsweise Sauerstoff. Oder das Zielgas ist ein Narkosemittel. Die Gasprobe stammt aus einem räumlichen Bereich, der auf das Zielgas zu überwachen ist.

In einer Anwendung führt ein Benutzer ein solches Gasmessgerät mit sich, während der Benutzer sich im räumlichen Bereich aufhält. Das mitgeführte Gasmessgerät warnt den Benutzer, wenn im räumlichen Bereich das Zielgas mit einer unzulässigen Konzentration auftritt. In einer anderen Anwendung lässt sich ein Gasmessgerät an einem Ort aufstellen und übermittelt eine gemessene Zielgas-Konzentration an einen räumlich entfernten Empfänger. Die Erfindung lässt sich in Kombination mit beiden Anwendungen verwenden.

Ein Ausgabeautomat für mobile Geräte und insbesondere für Gasmessgeräte wird in EP 3079109 B1 und US 10409237 B2 beschrieben. Der dort beschriebene Ausgabeautomat umfasst mehrere Lagerstationen 11 und mindestens eine Wartungsstation 14. Ein Prüfgas lässt sich von einer Gasquelle 40 über eine Gaszuführleitung 42 zur Wartungsstation 14 leiten. In der Wartungsstation 14 lässt sich ein Gasmessgerät 1 einsetzen und kalibrieren. Nach der Kalibrierung wird das Gasmessgerät 1 in eine Lagerstation 11 eingesetzt. Ein Benutzer kann einen Ausgabewunsch eingeben. Nachdem der Ausgabewunsch erkannt wurde, wird eine Gruppe von Gasmessgeräten ermittelt, und zwar abhängig von einem Verhältnis zwischen einem Wartungskennwert und einem Wartungsschwellwert oder Kalibrierungsschwellwert. Ein Gasmessgerät 1 der Gruppe wird ausgewählt, und die Lagerstation 11, in der sich das ausgewählte Gasmessgerät 1 befindet, wird entriegelt. Wenn der Benutzer das Gasmessgerät 1 zurückgeben will, setzt er es in die Wartungsstation 14 ein, wo es überprüft wird.

Der Erfindung liegt die Aufgabe zugrunde, einen Ausgabeautomaten sowie ein Ausgabe-Verfahren und ein Rückgabe-Verfahren bereitzustellen, die das Risiko eines Fehlers bei der Ausgabe bzw. der Rückgabe eines Gasmessgeräts verringern.

Die Aufgabe wird durch einen Ausgabeautomaten mit den Merkmalen des Anspruchs 1, durch ein Ausgabe-Verfahren mit den Merkmalen des Anspruchs 11 und durch ein Rückgabe-Verfahren mit den Merkmalen des Anspruchs 13 gelöst. Vorteilhafte Ausgestaltungen sind in den Unteransprüchen angegeben. Vorteilhafte Ausgestaltungen des erfindungsgemäßen Ausgabeautomaten sind, soweit sinnvoll, auch vorteilhafte Ausgestaltungen der erfindungsgemäßen Verfahren und umgekehrt.

Der Ausgabeautomat umfasst mindestens eine Aufnahmeeinheit, bevorzugt mindestens zwei Aufnahmeeinheiten, besonders bevorzugt mindestens fünf Aufnahmeeinheiten, insbesondere mindestens zehn Aufnahmeeinheiten. In die oder jede Aufnahmeeinheit lässt sich jeweils ein Gasmessgerät einsetzen, und es lässt sich wieder aus der Aufnahmeeinheit entnehmen. Was ein Gasmessgerät ist, wurde weiter oben beschrieben. Nachfolgend wird die Ausgestaltung mit einer Aufnahmeeinheit beschrieben, und bei Bedarf wird die Ausgestaltung mit mehreren Aufnahmeeinheiten beschrieben.

Der Ausgabeautomat umfasst einen Gaseinlass und eine Fluidführungs-Anordnung. Die Fluidführungs-Anordnung stellt eine Fluidverbindung zwischen dem Gaseinlass und einer Aufnahmeeinheit und damit zwischen dem Gaseinlass und einem Gasmessgerät, das in die Aufnahmeeinheit eingesetzt ist, her. Im Falle von mehreren Aufnahmeeinheiten stellt die Fluidführungs-Anordnung eine Fluidverbindung zwischen dem Gaseinlass einerseits und jeder Aufnahmeeinheit und damit mit jedem eingesetzten Gasmessgerät andererseits her.

Ein Verschluss des Ausgabeautomaten lässt sich relativ zur Aufnahmeeinheit zwischen einer verschließenden Position und einer geöffneten Position hin und her bewegen. In der verschließenden Position verhindert der Verschluss, dass sich ein Gasmessgerät aus der Aufnahmeeinheit entnehmen oder in die Aufnahmeeinheit einsetzen lässt. In der geöffneten Position gibt der Verschluss die Aufnahmeeinheit frei und ermöglicht dadurch diese Entnahme und dieses Einsetzen. Eine Arretiereinheit lässt sich in einen arretierenden Zustand und in einen freigebenden Zustand überführen. Bevorzugt umfasst die Arretiereinheit einen Arretierkörper, der relativ zum Verschluss beweglich ist, im arretierenden Zustand in eine Aufnahme des Verschlusses eingreift und dies im freigebenden Zustand nicht tut.

Wenn der Verschluss in der verschließenden Position und die Arretiereinheit im arretierenden Zustand sind, verhindert die Arretiereinheit, dass der Verschluss in die geöffnete Position überführt wird. Wenn die Arretiereinheit im freigebenden Zustand ist, ist der Verschluss dearretiert (entriegelt, freigegeben) und lässt sich in die geöffnete Position überführen, und zwar manuell und/oder mittels eines Stellelements, z.B. einer Feder.

Im Falle einer ersten und einer zweiten Aufnahmeeinheit umfasst der Ausgabeautomat einen ersten und einen zweiten Verschluss sowie eine erste und eine zweite Arretiereinheit. Jeder Verschluss lässt sich unabhängig vom anderen Verschluss in die verschließende Position und in die geöffnete Position überführen, und jede Arretiereinheit lässt sich unabhängig von der anderen Arretiereinheit in den arretierenden und in den freigebenden Zustand überführen. Der erste Verschluss und die erste Arretiereinheit sind der ersten Aufnahmeeinheit zugeordnet, der zweite Verschluss und die zweite Arretiereinheit der zweiten Aufnahmeeinheit. Das Entsprechende gilt für drei oder mehr Aufnahmeeinheiten. Allgemein ist jeder Aufnahmeeinheit jeweils ein Verschluss zugeordnet, und jedem Verschluss ist jeweils eine Arretiereinheit zugeordnet. Jeder Verschluss lässt sich unabhängig von jedem anderen Verschluss von der einen Position in die andere Position überführen, und jede Arretiereinheit lässt sich unabhängig von jeder anderen Arretiereinheit vom einen Zustand in den anderen Zustand überführen.

Das erfindungsgemäße Ausgabe-Verfahren wird unter Verwendung eines Ausgabeautomaten mit einer Aufnahmeeinheit, einem Verschluss und einer Arretiereinheit durchgeführt. Bevor das Ausgabe-Verfahren durchgeführt wird, ist folgende Situation hergestellt:
- Der Verschluss ist relativ zur Aufnahmeeinheit in der verschließenden Position.
- Die Arretiereinheit ist im arretierenden Zustand.

Bei mehreren Aufnahmeeinheiten ist diese Situation für jede Aufnahmeeinheit hergestellt. Bevor das Ausgabe-Verfahren durchgeführt wird, lässt sich also kein Gasmessgerät aus dem Ausgabeautomaten entnehmen - optional außer durch einen Servicetechniker mit besonderen Zugriffsrechten.

Eine Eingabeeinheit des Ausgabeautomaten vermag eine Benutzereingabe zu erfassen. Die erfasste Benutzereingabe umfasst mindestens die Information, dass die Ausgabe eines Gasmessgeräts gewünscht ist. Optional umfasst die Benutzereingabe weitere Informationen, insbesondere über eine gewünschte Eigenschaft oder geplante Verwendung eines auszugebenden Gasmessgeräts und / oder über die Person, die diese Benutzereingabe tätigt.

Der Ausgabeautomat ist dazu ausgestaltet, nach der Erfassung einer solchen Benutzereingabe die folgenden Schritte durchzuführen, und gemäß dem erfindungsgemäßen Ausgabe-Verfahren löst die Erfassung der Benutzereingabe die folgenden Schritte aus:
Eine signalverarbeitende Steuereinheit (control unit) des Ausgabeautomaten prüft, ob in die Aufnahmeeinheit ein Gasmessgerät eingesetzt ist und ob das eingesetzte Gasmessgerät verwendbar ist. Ein Gasmessgerät ist nur dann verwendbar, wenn es intakt ist. Anders ausgedrückt: Ein nicht intaktes Gasmessgerät ist nicht verwendbar. Ein Gasmessgerät ist intakt, wenn es die Konzentration eines vorgegebenen Zielgases in einer Gasprobe dergestalt zu messen vermag, dass die gemessene Zielgas-Konzentration von der tatsächlichen Zielgas-Konzentration um nicht mehr als eine vorgegebene absolute oder prozentuale Toleranz abweicht. Dies gilt für vorgegebene Einsatzbedingungen, insbesondere für jeweils einen vorgegebenen Bereich der Umgebungstemperatur, der Umgebungsfeuchte und des Umgebungsdrucks. Ausgestaltungen der Erfindung legen optionale zusätzliche Kriterien fest, die erfüllt sein müssen, damit ein intaktes Gasmessgerät verwendbar ist. Möglich ist, dass ein eingesetztes Gasmessgerät zwar intakt, aber aktuell oder für eine bestimmte Anwendung nicht verwendbar ist.

Anmerkung: Die Formulierung wird verwendet, dass ein Sensor eine physikalische Größe zu messen vermag, beispielsweise die Konzentration eines Zielgases in einer Gasprobe. Diese Formulierung bedeutet, dass der Sensor direkt die physikalische Größe zu messen vermag oder mindestens eine andere Größe, die mit der zu messenden Größe korreliert. Die oder eine gemessene andere Größe oder die Kombination der gemessenen anderen Größen zusammen sind daher ein Maß für die zu messende physikalische Größe. Die Messung liefert mindestens einen Wert für die gesuchte physikalische Größe.

Im Falle von mehreren Aufnahmeeinheiten sucht die Steuereinheit nach einem Gasmessgerät, das in eine Aufnahmeeinheit eingesetzt und verwendbar ist. Bei dieser Suche prüft die Steuereinheit mindestens einmal, ob ein eingesetztes Gasmessgerät verwendbar ist. Optional sucht die Steuereinheit unter mehreren eingesetzten Gasmessgeräte nach einem verwendbaren Gasmessgerät. Falls die Steuereinheit kein verwendbares Gasmessgerät findet, so bewirkt sie bevorzugt, dass eine entsprechende Fehlermeldung ausgegeben wird.

Möglich ist, dass die Steuereinheit mehrere eingesetzte und verwendbare Gasmessgeräte findet. Bei diesem Suchergebnis wählt bevorzugt die Steuereinheit ein verwendbares Gasmessgerät aus. In einer Ausgestaltung trifft die Steuereinheit diese Auswahl abhängig davon, wie zuverlässig die verwendbaren Gasmessgeräte die Zielgas-Konzentration zu messen vermögen, also wie genau die gemessene Zielgas-Konzentration von einer vorgegebenen und bekannten Zielgas-Konzentration einer Gasprobe abweicht. Beispielsweise wählt die Steuereinheit dasjenige Gasmessgerät aus, bei dem die Abweichung am geringsten ist. In der Regel umfasst jedes Gasmessgerät eine eigene Spannungsversorgungseinheit. Möglich ist, dass die Steuereinheit die Auswahl abhängig vom aktuellen Ladezustand dieser Spannungsversorgungseinheit trifft.

Möglich ist auch, dass die Steuereinheit dann, wenn mehrere eingesetzte und verwendbare Gasmessgeräte gefunden worden sind, folgenden Schritt auslöst: Auf einer Ausgabeeinheit wird für jedes eingesetzte und verwendbare Gasmessgerät jeweils eine Information ausgegeben. Der Benutzer wählt mithilfe der Ausgabeeinheit ein Gasmessgerät aus, und die Steuereinheit erfasst diese Benutzereingabe. Die Ausgabeeinheit kann ein Bestandteil des Ausgabeautomaten sein oder auch ein Bestandteil eines mobilen Geräts, beispielsweise eines Smartphones. Bei der zweiten Realisierungsform wird wenigstens zeitweise eine Datenverbindung zwischen dem Gerät und dem Ausgabeautomaten hergestellt.

Das Ergebnis, dass ein eingesetztes und verwendbares Gasmessgerät gefunden worden ist, löst folgenden weiteren Schritt aus: Die Steuereinheit bewirkt, dass die Arretiereinheit in den freigebenden Zustand überführt wird. Der Verschluss ist nunmehr dearretiert und lässt sich in die geöffnete Position bewegen, insbesondere manuell, oder öffnet sich von allein. Falls der Ausgabeautomat mehrere Aufnahmeeinheiten umfasst, so bewirkt die Steuereinheit Folgendes für diejenige Aufnahmeeinheit, in die das gefundene Gasmessgerät eingesetzt ist: Die Arretiereinheit, die dieser Aufnahmeeinheit zugeordnet ist, wird in den freigebenden Zustand überführt.

Dank der Erfindung lässt sich nur dann ein Gasmessgerät aus einer Aufnahmeeinheit entnehmen, wenn die Steuereinheit festgestellt hat, dass in diese Aufnahmeeinheit ein verwendbares Gasmessgerät eingesetzt ist. Insbesondere wird dadurch in vielen Fällen verhindert, dass der Benutzer ein Gasmessgerät entnimmt, das bereits als defekt erkannt worden ist oder dessen Zustand unbekannt ist oder das zwar intakt ist, aber für einen bestimmten Einsatz nicht verwendbar. Solange der Verschluss verschlossen und arretiert ist, wird verhindert oder wenigstens erschwert, dass eine unbefugte Person ein Gasmessgerät entnimmt.

Der erfindungsgemäße Ausgabeautomat erfordert oft weniger Platz als ein Ausgabeautomat, der so wie in EP 3079109 B1 und US 10409237 B2 beschrieben aufgebaut ist. Denn der erfindungsgemäße Ausgabeautomat umfasst nur eine Art von Aufnahmeeinheiten, wobei die oder jede Aufnahmeeinheit sowohl dafür ausgestaltet ist, ein Gasmessgerät zur Ausgabe bereitzuhalten, als auch dafür ausgestaltet ist, die Überprüfung dieses Gasmessgeräts zu ermöglichen, nämlich indem das Gasmessgerät eine Zielgas-Konzentration in einer zugeführten Gasprobe misst, wobei bevorzugt die Zielgas-Konzentration in der Gasprobe bekannt ist. Der in EP 3079109 B1 und US 10409237 B2 beschriebene Ausgabeautomat umfasst hingegen Lagerstationen, in denen jeweils ein Gasmessgerät zur Ausgabe bereitgehalten wird, und mindestens eine Wartungsstation, in der ein Gasmessgerät kalibriert wird.

Erfindungsgemäß stellt die Fluidführungs-Anordnung eine Fluidverbindung zwischen dem Gaseinlass und der Aufnahmeeinheit her. Der Ausgabeautomat ermöglicht es daher, dass eine Gasprobe zu einem eingesetzten Gasmessgerät geleitet wird. Diese Gasprobe weist das Zielgas mit einer bekannten und vorgegebenen Konzentration auf. Ermöglicht wird, dass das eingesetzte Gasmessgerät die Zielgas-Konzentration in der Gasprobe misst und dass der Ausgabeautomat die gemessene Zielgas-Konzentration mit der vorgegebenen Zielgas-Konzentration vergleicht. Die Steuereinheit entscheidet abhängig von diesem Vergleich, ob das eingesetzte Gasmessgerät intakt ist oder nicht. Erfindungsgemäß ist eine Fluidverbindung zwischen dem Gaseinlass und einem eingesetzten Gasmessgerät hergestellt. Dieses Merkmal ermöglicht es, dass ein eingesetztes Gasmessgerät eine Zielgas-Konzentration in einer Gasprobe misst, wobei diese Gasprobe vom Gaseinlass zum eingesetzten Gasmessgerät geflossen ist. Diese Messung und dieser Vergleich können vorab durchgeführt werden, also bevor ein Benutzer eine Ausgabe eines Gasmessgerätes wünscht.

Die Entscheidung, ob ein Gasmessgerät aktuell verwendbar ist oder nicht, lässt sich auf Basis einer aktuellen Messung und eines aktuellen Vergleichs durchführen. Nicht erforderlich ist es, auf abgespeicherte Daten zurückzugreifen, welche veraltet sein können. Dank der Erfindung wird das Risiko verringert, dass der Ausgabeautomat ein Gasmessgerät ausgibt, welches eine Zielgas-Konzentration aktuell nicht korrekt zu messen vermag. Durch eine falsche Messung einer Zielgas-Konzentration könnte ein Benutzer gefährdet werden. Dank der Erfindung ist es nicht erforderlich, ein Gasmessgerät zu entnehmen, um es zu überprüfen. Vielmehr lässt sich oft die Überprüfung durchführen, während das Gasmessgerät eingesetzt ist.

Der erfindungsgemäße Ausgabeautomat lässt sich dank der Erfindung gleichzeitig als Prüfstation für das oder jedes eingesetzte Gasmessgerät verwenden. Möglich, aber dank der Erfindung nicht erforderlich ist es, ein eingesetztes Gasmessgerät aus der Aufnahmeeinheit zu entnehmen, um es zu überprüfen. Außerdem ist dank der Erfindung Folgendes nicht erforderlich: Ein Gasmessgerät, das in eine Aufnahmeeinheit mit einer Fluidverbindung zum Gaseinlass eingesetzt ist, wird überprüft oder kalibriert und dann in eine andere Aufnahmeeinheit eingesetzt, wo es für eine Ausgabe bereitgehalten wird. Vielmehr ermöglicht es die Erfindung, ein Gasmessgerät in derselben Aufnahmeeinheit sowohl zu überprüfen als auch für eine Ausgabe bereitzuhalten.

In vielen Fällen lässt sich eine bereits vorhandene Prüfstation so umbauen, dass nach dem Umbau ein erfindungsgemäßer Ausgabeautomat bereitgestellt wird. In der Regel umfasst eine solche Prüfstation bereits mindestens eine Aufnahmeeinheit, den Gaseinlass und die Fluidführungs-Anordnung sowie eine Steuereinheit. In vielen Fällen reicht es aus, mindestens einen Verschluss und mindestens eine Arretiereinheit zu ergänzen und eine Software auf der Steuereinheit anzupassen.

Bevorzugt hat die Steuereinheit Lesezugriff auf einen Datenspeicher. In diesem Datenspeicher ist für jedes eingesetzte Gasmessgerät die Information abgespeichert, ob dieses Gasmessgerät aktuell intakt ist oder nicht. Diese Information resultiert beispielsweise aus einem Prüfvorgang, bei dem ein Prüfgas mit einer bekannten Zielgas-Konzentration zum eingesetzten Gasmessgerät geleitet wird und die Zielgas-Konzentration, die das Gasmessgerät misst, mit der bekannten Zielgas-Konzentration verglichen wird. Die Information kann auch von einem Wartungstechniker eingegeben worden sein. Der Datenspeicher kann ein Bestandteil des Ausgabeautomaten oder auch ein Bestandteil des Gasmessgeräts oder räumlich entfernt angeordnet sein.

Erfindungsgemäß lässt sich ein Gasmessgerät aus der Aufnahmeeinheit entnehmen, wenn der Verschluss in der geöffneten Position ist. Der Verschluss lässt sich nur dann in die geöffnete Position bewegen, wenn die Arretiereinheit im freigebenden Zustand ist. In einer Ausgestaltung bewegt ein Benutzer den Verschluss in die geöffnete Position. In einer anderen Ausgestaltung umfasst die Aufnahmeeinheit ein Öffnungselement. Dieses Öffnungselement vermag den Verschluss aus der verschließenden in die geöffnete Position zu bewegen. Bevorzugt arretiert die Arretiereinheit den geschlossenen Verschluss gegen die Kraft des Öffnungselements. Diese Ausgestaltung bewirkt Folgendes: Sobald die Arretiereinheit in den freigebenden Zustand überführt, also dearretiert, worden ist, öffnet das Öffnungselement automatisch den Verschluss. Der Verschluss springt also von allein auf. Diese Ausgestaltung erspart die Notwendigkeit, einem Benutzer auf einem separaten Anzeigeelement anzuzeigen, dass sich nunmehr aus der Aufnahmeeinheit ein verwendbares Gasmessgerät entnehmen lässt. Außerdem ist es nicht erforderlich, dass der Benutzer den Verschluss manuell öffnet.

Im Falle von mehreren Aufnahmeeinheiten umfasst bevorzugt jede Aufnahmeeinheit jeweils ein Öffnungselement, welches den zugeordneten Verschluss in die geöffnete Position zu bewegen vermag. Diese Ausgestaltung erspart zusätzlich die Notwendigkeit, einem Benutzer anzuzeigen, in welcher Aufnahmeeinheit sich ein verwendbares Gasmessgerät befindet, das sich jetzt entnehmen lässt.

Bevorzugt ist das oder jedes Öffnungselement als ein rein mechanisches Bauteil ausgestaltet, benötigt also keine Versorgung mit elektrischer oder pneumatischer oder hydraulischer Energie und auch keine Ansteuerung. Insbesondere umfasst das oder jedes Öffnungselement jeweils eine Feder.

Möglich ist, dass sich der Verschluss durch eine lineare Bewegung relativ zur Aufnahmeeinheit von der einen Position in die andere Position bewegen, also verschieben lässt. In einer bevorzugten Ausgestaltung lässt sich der Verschluss hingegen durch eine Drehung von der einen Position in die andere Position bewegen, also drehen. Bevorzugt ist die Schwerkraft bestrebt, den Verschluss in die verschließende Position zu drehen. Im Falle von mehreren Aufnahmeeinheiten und somit mehreren Verschlüssen sind bevorzugt diese Verschlüsse dergestalt nebeneinander angeordnet, dass sich alle Verschlüsse um dieselbe Drehachse drehen lassen, und zwar unabhängig voneinander.

Weiter oben wurde eine Ausgestaltung beschrieben, bei der ein Öffnungselement den Verschluss in die geöffnete Position zu bewegen vermag. Bevorzugt ist der Verschluss drehbar angeordnet, und das Öffnungselement vermag den Verschluss in die geöffnete Position zu drehen, bevorzugt gegen die Schwerkraft.

In vielen Fällen umfasst ein Gasmessgerät eine eigene Spannungsversorgungseinheit, insbesondere mindestens eine wiederaufladbare Batterie. In einer Ausgestaltung umfasst die Aufnahmeeinheit eine elektrische Kontaktierung. Im Falle von mehreren Aufnahmeeinheiten umfasst bevorzugt jede Aufnahmeeinheit jeweils eine elektrische Kontaktierung.

In einer Realisierungsform wird die Spannungsversorgungseinheit aufgeladen oder lässt sich aufladen, solange das Gasmessgerät in die Aufnahmeeinheit eingesetzt ist. Das Aufladen wird mithilfe der elektrischen Kontaktierung durchgeführt. Der Ausgabeautomat ist mit einem stationären Spannungsversorgungsnetz verbunden und / oder umfasst eine eigene Spannungsversorgungseinheit. Dank dieser Realisierungsform ist es nicht erforderlich, das Gasmessgerät zu entnehmen, um seine Spannungsversorgungseinheit aufzuladen. Dies spart Zeit ein, verglichen mit einer Ausgestaltung, bei der das Gasmessgerät aus dem Ausgabeautomaten entnommen und in eine separate Ladestation eingesetzt werden müsste.

Bevorzugt umfasst der Ausgabeautomat einen Spannungssensor. Falls ein Gasmessgerät in die Aufnahmeeinheit eingesetzt ist, so ist mittels der elektrischen Kontaktierung eine elektrische Verbindung zwischen dem Spannungssensor und der Spannungsversorgungseinheit des eingesetzten Gasmessgeräts hergestellt oder lässt sich herstellen. Der Spannungssensor vermag den aktuellen Ladezustand der Spannungsversorgungseinheit zu messen. Es ist auch möglich, dass der Ausgabeautomat den aktuellen Ladezustand der Spannungsversorgungseinheit von einem Spannungssensor, der ein Teil des Gasmessgeräts ist, zu erfassen vermag.

Bei mehreren Aufnahmeeinheiten sind folgende Alternativen möglich: In einer Alternative ist allen Aufnahmeeinheiten derselbe Spannungssensor zugeordnet. In einer anderen Alternative ist jeder Aufnahmeeinheit jeweils ein eigener Spannungssensor zugeordnet.

Erfindungsgemäß prüft die Steuereinheit, ob ein eingesetztes Gasmessgerät verwendbar ist. Gemäß der Ausgestaltung mit der elektrischen Kontaktierung entscheidet die Steuereinheit dann, dass das eingesetzte Gasmessgerät aktuell nicht verwendbar ist, wenn der gemessene Ladezustand kleiner ist als eine Ladezustands-Schranke. In einer Realisierungsform ist diese Ladezustands-Schranke fest vorgegeben. Nachfolgend wird eine abweichende Realisierungsform beschrieben.

Erfindungsgemäß vermag die Eingabeeinheit eine Benutzereingabe zu erfassen. In einer Ausgestaltung umfasst diese Benutzereingabe zusätzlich eine Information, wie lange ein geplanter Einsatz eines auszugebenden Gasmessgeräts gewünscht ist. Während dieses Einsatzes ist es in der Regel nicht möglich oder wenigstens nicht gewünscht, die Spannungsversorgungseinheit aufzuladen oder auszutauschen. Die Steuereinheit ist dazu ausgestaltet, abhängig vom gemessenen Ladezustand vorherzusagen, wie lange die Spannungsversorgungseinheit des eingesetzten Gasmessgeräts das Gasmessgerät mit elektrischer Energie zu versorgen vermag. Gemäß dieser Ausgestaltung bewertet die Steuereinheit das eingesetzte Gasmessgerät dann als verwendbar, wenn es intakt ist und wenn seine Spannungsversorgungseinheit das Gasmessgerät mindestens für die erfasste gewünschte Zeitdauer mit elektrischer Energie zu versorgen vermag. Die Ladezustands-Schranke hängt also von der gewünschten Zeitdauer der Benutzung ab.

Um die Vorhersage zu treffen, verwendet die Steuereinheit ein Signal des Spannungssensors, also den gemessenen aktuellen Ladezustand, sowie einen vorgegebenen oder vorab gemessenen Wert dafür, wie viel elektrische Energie das Gasmessgerät pro Zeiteinheit verbraucht.

Dank dieser Ausgestaltung wird in vielen Fällen ein eingesetztes Gasmessgerät häufiger als verwendbar bewertet, als wenn nur ein vollständig aufgeladenes Gasmessgerät verwendbar wäre.

Gemäß der gerade beschriebenen Ausgestaltung bewertet die Steuereinheit ein eingesetztes Gasmessgerät nur dann als verwendbar, wenn es intakt ist und wenn der gemessene Ladezustand seiner Spannungsversorgungseinheit oberhalb einer vorgegebenen oder von der Benutzereingabe abhängenden Ladezustands-Schranke liegt. In einer Fortbildung dieser Ausgestaltung ist die Steuereinheit wie folgt ausgestaltet: Falls der gemessene Ladezustand kleiner ist als die Ladezustands-Schranke, sagt die Steuereinheit vorher, wann der Ladezustand die Ladezustands-Schranke erreicht haben wird. Bei dieser Fortbildung wird die Spannungsversorgungseinheit aufgeladen, solange das Gasmessgerät eingesetzt ist. Um diese Vorhersage zu treffen, verwendet die Steuereinheit den gemessenen aktuellen Ladezustand sowie einen vorgegebenen oder vorab gemessenen Wert dafür, wie schnell der Ladezustand beim Aufladen steigt. Dieser Wert kann vom Typ und / oder dem Alter des Gasmessgeräts abhängen. Bevorzugt gibt der Ausgabeautomat die vorhergesagte Zeitspanne, die zum Aufladen erforderlich ist, in einer von einem Menschen wahrnehmbaren Form aus.

Bevorzugt umfasst der Ausgabeautomat mehrere Aufnahmeeinheiten. Möglich ist, dass die Steuereinheit mehrere eingesetzte und verwendbare Gasmessgeräte findet. In einer Ausgestaltung wählt die Steuereinheit dasjenige Gasmessgerät aus, dessen Spannungsversorgungseinheit den höchsten Ladezustand aufweist oder das Gasmessgerät am längsten mit elektrischer Energie zu versorgen vermag. Oder die Steuereinheit wendet ein Auswahlkriterium an, welches von der Genauigkeit der Zielgas-Messung und vom gemessenen Ladezustand abhängt. Die Steuereinheit bewirkt, dass sich das dergestalt ausgewählte Gasmessgerät aus dem Ausgabeautomaten entnehmen lässt.

In einer Realisierungsform wird der Spannungssensor zusätzlich dafür verwendet, einen Fehler zu detektieren. Der Spannungssensor misst wiederholt den aktuellen Ladezustand eines eingesetzten Gasmessgeräts, optional mehrerer Gasmessgeräte, und die Steuereinheit leitet einen zeitlichen Verlauf des Ladezustands her. Falls dieser Ladezustand über einen ausreichend langen Zeitraum unverändert bleibt, so kann dies insbesondere eine der folgenden drei Ursachen haben:
- Die Spannungsversorgungseinheit ist vollständig aufgeladen (eine gewünschte Situation).
- Das Gasmessgerät ist nicht korrekt in die Aufnahmeeinheit eingesetzt, und daher wird die Spannungsversorgungseinheit nicht elektrisch kontaktiert (ein Fehler).
- Die Spannungsversorgungseinheit ist defekt, oder der Ausgabeautomat vermag die Spannungsversorgungseinheit nicht mit elektrischer Energie zu versorgen (ein Fehler).

Erfindungsgemäß vermag die Eingabeeinheit eine Benutzereingabe zu erfassen. In einer Ausgestaltung umfasst die erfasste Benutzereingabe zusätzlich eine Information über eine gewünschte Eigenschaft eines Gasmessgeräts. Beispiele für eine gewünschte Eigenschaft sind die folgenden:
- Das Gasmessgerät soll ein bestimmtes Zielgas zu messen vermögen, optional mehrere Zielgase, die gleichzeitig auftreten können.
- Das Gasmessgerät soll die Konzentration des Zielgases bei einer vorgegebenen Umgebungsbedingung zuverlässig messen können. Die vorgegebene Umgebungsbedingung spezifiziert beispielsweise einen Wertebereich für die Umgebungstemperatur oder für die Umgebungsfeuchte oder für den Umgebungsdruck. Beispielsweise soll das Gasmessgerät die Zielgas-Konzentration auch bei einer sehr hohen Umgebungstemperatur oder einer sehr großen Umgebungsfeuchte oder in einer Röhre zu messen vermögen.
- Das Gasmessgerät soll nicht schwerer und / oder nicht größer sein als eine vorgegebene obere Schranke für das Gewicht oder für eine Abmessung.
- Das Gasmessgerät soll einen Alarm auf eine bestimmte Weise ausgeben können, beispielsweise haptisch (durch Vibrationen).
- Das Gasmessgerät soll eine beleuchtete Anzeigefläche aufweisen, damit eine Anzeige auch im Dunkeln abgelesen werden kann.
- Das Gasmessgerät soll sprachliche Eingaben und / oder Ausgaben in einer bestimmten Sprache erfassen bzw. erzeugen können.

Gemäß der gerade beschriebenen Ausgestaltung bewertet die Steuereinheit ein eingesetztes Gasmessgerät nur dann als verwendbar, wenn es intakt ist und zusätzlich die oder jede gewünschte Eigenschaft aufweist. Nur dann, wenn es die oder jede gewünschte Eigenschaft aufweist, wird die Arretiereinheit in den freigebenden Zustand überführt, sodass sich das Gasmessgerät entnehmen lässt. Diese Ausgestaltung verhindert folgende unerwünschte Situation: Ein Benutzer entnimmt dem Ausgabeautomaten ein Gasmessgerät, das zwar intakt ist, sich aber für den geplanten Einsatz überhaupt nicht oder nicht mit ausreichender Zuverlässigkeit oder Ergonomie verwenden lässt.

Bevorzugt umfasst die erfasste Benutzereingabe zusätzlich eine Kennzeichnung des Benutzers, der dem Ausgabeautomaten ein Gasmessgerät entnehmen will. Mithilfe dieser Kennzeichnung überprüft die Steuereinheit des Ausgabeautomaten, ob der Benutzer berechtigt ist, ein Gasmessgerät zu entnehmen. Insbesondere überprüft die Steuereinheit, ob der Benutzer überhaupt autorisiert ist, und / oder ob der Benutzer dafür qualifiziert ist, ein Gasmessgerät mit einer bestimmten gewünschten Eigenschaft zu verwenden. Die Kennzeichnung kann beispielsweise eine Benutzerkennung und ein Passwort umfassen. Möglich ist, dass die Eingabeeinheit ein Benutzer-Lesegerät umfasst, das einen Chip auf einer Chipkarte auszulesen vermag, oder einen Sensor für ein biometrisches Merkmal eines Benutzers.

Die nachfolgend beschriebene Fortbildung dieser Ausgestaltung lässt sich mit der bereits beschriebenen Ausgestaltung kombinieren, bei der die erfasste Benutzereingabe eine gewünschte Eigenschaft eines Gasmessgeräts spezifiziert. Gemäß dieser Fortbildung muss ein Benutzer eine bestimmte Qualifikation aufweisen, um ein Gasmessgerät mit dieser erfassten Eigenschaft benutzen zu dürfen. In einem Datenspeicher ist das jeweilige Qualifikationsprofil einer Menge von registrierten Benutzern hinterlegt. Nachdem die Eingabeeinheit eine Kennzeichnung des Benutzers erfasst hat, ermittelt die Steuereinheit das hinterlegte Qualifikationsprofil dieses Benutzers. Die Steuereinheit bewertet ein eingesetztes Gasmessgerät nur dann als verwendbar, wenn es intakt ist und wenn der registrierte und erkannte Benutzer gemäß dem ermittelten Qualifikationsprofil das Gasmessgerät benutzen darf.

Die beiden gerade beschriebenen Ausgestaltungen lassen sich miteinander kombinieren. Die Steuereinheit bewertet ein eingesetztes Gasmessgerät gemäß dieser Kombination nur dann als verwendbar, wenn es die oder jede Eigenschaft aufweist, die gemäß der Benutzereingabe erforderlich oder gewünscht ist, und wenn zusätzlich der registrierte und erkannte Benutzer dieses Gasmessgerät benutzen darf. Möglich ist, dass das Qualifikationsprofil eines Benutzers spezifiziert, welche Eigenschaft ein Gasmessgerät hat oder haben kann, welches dieser Benutzer verwenden darf.

Erfindungsgemäß stellt die Fluidführungs-Anordnung eine Fluidverbindung zwischen dem Gaseinlass und einem eingesetzten Gasmessgerät her. In einer Ausgestaltung umfasst die Aufnahmeeinheit ein Kontaktelement. Relativ zu einem eingesetzten Gasmessgerät lässt sich das Kontaktelement zwischen einer kontaktierenden und einer beabstandeten Position hin und her bewegen. Die gerade beschriebene Fluidverbindung mit dem Gaseinlass ist gemäß dieser Ausgestaltung nur dann hergestellt, wenn das Kontaktelement in der kontaktierenden Position ist, und führt durch das Kontaktelement. Ansonsten ist diese Fluidverbindung unterbrochen. Im Falle von mehreren Aufnahmeeinheiten umfasst bevorzugt jede Aufnahmeeinheit jeweils ein Kontaktelement, und jedes Kontaktelement lässt sich unabhängig von dem oder jedem anderen Kontaktelement von der einen Position in die andere Position bewegen.

Ein Vorteil des beweglichen Kontaktelements verglichen mit einer Ausgestaltung ohne Kontaktelement ist das folgende: In der Regel lässt sich das eingesetzte Gasmessgerät nur dann oder wenigstens dann leichter aus der Aufnahmeeinheit entnehmen und wieder einsetzen, wenn das Kontaktelement in der beabstandeten Position ist.

In einer Ausgestaltung umfasst das Kontaktelement ein Ventil. Die Fluidverbindung ist hergestellt, wenn das Ventil geöffnet ist. Der Vorgang, dass das Kontaktelement in die kontaktierende Position bewegt wird, löst bevorzugt den Vorgang aus, dass das Ventil geöffnet wird. Umgekehrt löst bevorzugt der Vorgang, dass das Kontaktelement aus der kontaktierenden Position heraus bewegt wird, den Vorgang aus, dass das Ventil geschlossen wird.

Bevorzugt befindet sich das Kontaktelement zwischen dem Verschluss und einem Bereich der Aufnahmeeinheit, wobei der Bereich beispielsweise eine Ausbuchtung ist, in die sich ein Gasmessgerät einsetzen lässt. Wenn in die Aufnahmeeinheit ein Gasmessgerät eingesetzt ist und der Verschluss in der verschließenden Position ist, befindet sich das Kontaktelement zwischen dem Verschluss und dem Gasmessgerät.

In einer bevorzugten Realisierungsform sind sowohl der Verschluss als auch das Kontaktelement relativ zur Aufnahmeeinheit drehbar angeordnet. Die beiden Drehachsen dieser drehbaren Anordnung sind bevorzugt parallel zueinander oder stimmen miteinander überein.

Besonders bevorzugt ist das Kontaktelement dergestalt mit dem Verschluss mechanisch gekoppelt, dass folgende Wirkung erzielt wird: Eine Bewegung des Verschlusses von der geöffneten Position in die verschließende Position bewirkt, dass das Kontaktelement von der beabstandeten in die kontaktierende Position überführt wird. Dank dieser Realisierungsform ist nur eine einzige Handlung erforderlich, um zu bewirken, dass der Verschluss die Aufnahmeeinheit verschließt und dass eine Fluidverbindung zwischen dem oder einem Gaseinlass und einer Messkammer des eingesetzten Gasmessgeräts hergestellt wird. Insbesondere braucht ein Benutzer, der ein Gasmessgerät zurückgeben will, nur eine einzige Handlung vorzunehmen.

In einer Ausgestaltung lässt sich ein Betätigungselement des Ausgabeautomaten manuell betätigen. Das Betätigungselement ist mit der Arretiereinheit dergestalt verbunden, dass folgende Wirkungskette realisiert wird: Eine Betätigung des Betätigungselements bewirkt, dass die Arretiereinheit in den freigebenden Zustand überführt wird. Im Falle von mehreren Aufnahmeeinheiten und damit mehreren Arretiereinheiten sind folgende alternative Realisierungsformen möglich:
- Dasselbe Betätigungselement ist mit allen Arretiereinheiten dergestalt verbunden, dass durch eine Betätigung des Betätigungselements alle Arretiereinheiten in den freigebenden Zustand überführt werden.
- Mit jeder Arretiereinheit ist jeweils ein Betätigungselement verbunden. Jedes Betätigungselement lässt sich unabhängig von dem oder jedem anderen Betätigungselement betätigen. Eine Arretiereinheit wird durch eine Betätigung des verbundenen Betätigungselements in den freigebenden Zustand überführt. Dadurch lässt sich jede Arretiereinheit unabhängig von der oder jeder anderen Arretiereinheit in den freigebenden Zustand überführen.

Die Ausgestaltung mit dem Betätigungselement ermöglicht es insbesondere einem Wartungstechniker, die Arretiereinheit in den freigebenden Zustand zu überführen und den Verschluss zu öffnen, und zwar unabhängig vom Zustand des eingesetzten Gasmessgeräts. Insbesondere wird der Wartungstechniker in die Lage versetzt, ein defektes Gasmessgerät zu entnehmen. In einer Ausgestaltung muss eine Person sich vorab autorisieren, bevor sie das Betätigungselement betätigen kann.

Bevorzugt ist das Betätigungselement ein rein mechanisches Teil. "Rein mechanisch" bedeutet, dass das Betätigungselement keine elektrische oder pneumatische oder hydraulische Energie und auch keine Ansteuerung benötigt. Bei dieser Ausgestaltung lässt der Verschluss sich in der Regel auch dann öffnen, wenn ein Defekt aufgetreten ist oder wenn eine Versorgung des Ausgabeautomaten mit elektrischer Energie ausgefallen oder unterbrochen ist. Beispielsweise umfasst das Betätigungselement einen Hebel und / oder ein Rad.

In einer Realisierungsform umfasst das mechanische Betätigungselement ein mechanisches Schloss und einen passenden Schlüssel. Das mechanische Schloss ist in den Verschluss oder in die Arretiereinheit eingelassen. Das Schloss lässt sich mithilfe des passenden Schlüssels wahlweise in einen verschließenden Zustand und in einen freigebenden Zustand überführen. Das Schloss ist wie folgt mit der zugeordneten Arretiereinheit verbunden, bevorzugt rein mechanisch verbunden: Eine Überführung des mechanischen Schlosses in den freigebenden Zustand bewirkt, dass die Arretiereinheit in den freigebenden Zustand überführt wird. Verglichen mit einem Hebel oder einem Rad verringert die Ausgestaltung mit dem Schloss und dem Schlüssel das Risiko, dass eine unbefugte Person den Verschluss öffnet und ein eingesetztes Gasmessgerät entnimmt.

In einer Fortbildung dieser Realisierungsform ist das mechanische Schloss wie folgt mit der Arretiereinheit gekoppelt: Eine Überführung der Arretiereinheit in den arretierenden Zustand bewirkt, dass das Schloss in den verschließenden Zustand überführt wird. Oder umgekehrt bewirkt eine Überführung des Schlosses in den verschließenden Zustand, dass die Arretiereinheit in den arretierenden Zustand überführt wird. Diese beiden Wirkungen treten mindestens dann auf, wenn der zugeordnete Verschluss in der verschließenden Position ist.

Möglich ist auch, dass das Betätigungselement ein Benutzer-Lesegerät umfasst, mit dem ein Benutzer sich autorisieren kann. Das Benutzer-Lesegerät erfasst beispielsweise ein Passwort oder ein biometrisches Merkmal des Benutzers. Bei erfolgreicher Autorisierung wird die oder jede Arretiereinheit automatisch in den freigebenden Zustand überführt.

Die Situation könnte auftreten, dass ein Benutzer versucht, einen Verschluss einer Aufnahmeeinheit zu öffnen, obwohl die Arretiereinheit für diesen Verschluss im arretierenden Zustand ist. In einer Ausgestaltung umfasst die Aufnahmeeinheit zusätzlich einen Sensor, beispielsweise einen Kraftsensor oder einen Positionssensor. Dieser Sensor vermag das Ereignis zu detektieren, dass versucht wird, den arretierten Verschluss zu öffnen. Falls der Sensor dieses Ereignis detektiert hat, so bewirkt die Steuereinheit, dass ein Alarm ausgegeben wird.

In einer Ausgestaltung umfasst der Ausgabeautomat eine Kamera und eine signalverarbeitende Bildauswerteeinheit. Die Kamera vermag von einem Gasmessgerät ein Bild zu erzeugen. In einer Realisierungsform ist die Kamera so positioniert, dass Folgendes ermöglicht wird: Die Kamera vermag ein Bild von einem Gasmessgerät, das in eine Aufnahmeeinheit eingesetzt ist, zu erzeugen. In einer bevorzugten Realisierungsform ist die Kamera hingegen so positioniert, dass Folgendes ermöglicht wird: Die Kamera vermag ein Bild von einem Gasmessgerät, das sich außerhalb des Ausgabeautomaten befindet, zu erzeugen. Bevorzugt umfasst der Ausgabeautomat eine Unterlage, beispielsweise eine Schale oder Platte, und die Kamera vermag ein Bild von einem Gasmessgerät auf der Unterlage zu erzeugen. Die Bildauswerteeinheit vermag das Bild oder die Bilder eines Gasmessgeräts auszuwerten und dadurch automatisch zu entscheiden, ob das gezeigte Gasmessgerät eine optisch erkennbare Beschädigung aufweist oder nicht, und bei einer Beschädigung bevorzugt, welche Beschädigung. Die optisch erkennbare Beschädigung ist insbesondere eine mechanische Beschädigung oder eine Verschmutzung.

Die Ausgestaltung mit der Kamera und der Bildauswerteeinheit ermöglicht es, automatisch zu überprüfen, ob ein ausgegebenes Gasmessgerät beschädigt ist. Falls die Kamera ein Bild eines Gasmessgeräts außerhalb des Ausgabeautomaten zu erzeugen vermag, so ist es möglich, vor der Rückgabe des Gasmessgeräts zu überprüfen, ob es in optisch erkennbarer Weise beschädigt ist oder nicht.

Die Erfindung betrifft weiterhin ein System. Dieses System umfasst einen erfindungsgemäßen Ausgabeautomaten, ein Gasmessgerät, eine Fluidführungseinheit und einen Behälter mit einem Prüfgas. Das Gasmessgerät ist in die Aufnahmeeinheit eingesetzt. Im Falle von mehreren Aufnahmeeinheiten ist das Gasmessgerät in eine Aufnahmeeinheit eingesetzt, und bevorzugt ist in eine weitere Aufnahmeeinheit ein weiteres Gasmessgerät eingesetzt. Eine Messkammer des oder jedes Gasmessgeräts vermag eine Gasprobe aufzunehmen, und das Gasmessgerät misst die Konzentration eines Zielgases in der aufgenommenen Gasprobe.

Das Prüfgas im Behälter weist ein Zielgas mit einer vorgegebenen Zielgas-Konzentration auf. Die Fluidführungseinheit vermag eine Fluidverbindung zwischen dem Behälter mit dem Prüfgas und dem Gaseinlass des Ausgabeautomaten herzustellen. In einer Ausgestaltung des Systems ist die Fluidführungseinheit dauerhaft mit dem Ausgabeautomaten verbunden, in einer anderen Ausgestaltung lösbar. Die Fluidführungs-Anordnung verbindet den Gaseinlass mit der Messkammer des eingesetzten Gasmessgeräts. In einer Realisierungsform ist diese Fluidverbindung nur dann hergestellt, wenn das oben beschriebene bewegliche Kontaktelement der Aufnahmeeinheit in der kontaktierenden Position ist.

Das erfindungsgemäße System ist dazu ausgestaltet, folgende Schritte durchzuführen:
- Das System bewirkt oder ermöglicht, dass eine Gasprobe mit dem Prüfgas vom Behälter zur Aufnahmeeinheit und weiter zum eingesetzten Gasmessgerät fließt.
- Die Steuereinheit bewirkt, dass das eingesetzte Gasmessgerät die Zielgas-Konzentration in der Gasprobe misst.
- Die gemessene Zielgas-Konzentration wird mit der vorgegebenen Zielgas-Konzentration verglichen, und zwar bevorzugt durch den Ausgabeautomaten.
- Die Steuereinheit entscheidet, dass das Gasmessgerät nicht intakt ist, wenn die gemessene Zielgas-Konzentration von der vorgegebenen Zielgas-Konzentration um mehr als die Toleranz abweicht.

In einer Ausgestaltung ist das System dazu ausgestaltet, mindestens einmal ein Prüfverfahren durchzuführen. Bevorzugt wird das Prüfverfahren wiederholt durchgeführt, beispielsweise mit einer vorgegebenen Frequenz oder jedes Mal dann, wenn der Ausgabeautomat eingeschaltet wird. Möglich ist auch, dass das Prüfverfahren als Reaktion auf eine erfasste entsprechende Benutzereingabe durchgeführt wird. Bei der oder jeder Durchführung des Prüfverfahrens werden die gerade beschriebenen Schritte durchgeführt, und die Steuereinheit entscheidet, ob das eingesetzte Gasmessgerät intakt ist oder nicht. Bei mehreren Aufnahmeeinheiten entscheidet die Steuereinheit für das oder jedes eingesetzte Gasmessgerät, ob es intakt ist oder nicht. Bevorzugt speichert die Steuereinheit das jeweilige Ergebnis jeder Prüfung ab und verwendet das abgespeicherte Ergebnis, wenn die Ausgabe eines Gasmessgeräts gewünscht wird.

Im Falle von mehreren Aufnahmeeinheiten wird in einer Ausgestaltung das Prüfverfahren für jedes Gasmessgerät durchgeführt, das in eine Aufnahmeeinheit eingesetzt ist. In einer anderen Ausgestaltung wird zuvor mindestens eine Aufnahmeeinheit ausgewählt, beispielsweise als Reaktion auf eine entsprechende Benutzereingabe oder automatisch abhängig davon, wann das Gasmessgerät in dieser Aufnahmeeinheit das letzte Mal überprüft worden ist. Das Prüfverfahren wird für das oder jedes Gasmessgerät durchgeführt, das in jeweils eine ausgewählte Aufnahmeeinheit eingesetzt ist.

Die gerade beschriebene Ausgestaltung ermöglicht es, zu prüfen, wie genau ein eingesetztes Gasmessgerät die Konzentration eines Zielgases zu messen vermag. In einer Ausgestaltung umfasst das System zwei Behälter mit zwei verschiedenen Prüfgasen, wobei die jeweilige Konzentration jedes Prüfgases im Behälter bekannt ist. Eine pneumatische Weiche mit zwei Eingängen und einem Ausgang lässt sich ansteuern und leitet je nach Ansteuerung Prüfgas aus dem einen oder Prüfgas aus dem anderen Behälter zum Ausgabeautomaten. Diese Ausgestaltung ermöglicht es, zu prüfen, mit welcher Genauigkeit das Gasmessgerät die jeweilige Konzentration der beiden Zielgase zu messen vermag. Diese Ausgestaltung lässt sich auf mehr als zwei Zielgase ausweiten.

In einer Ausgestaltung vermag die Eingabeeinheit des Ausgabeautomaten eine Benutzereingabe zu erfassen. Diese Benutzereingabe spezifiziert, dass ein Gasmessgerät, das in die Aufnahmeeinheit eingesetzt worden ist, überprüft werden soll, nämlich durch das gerade beschriebene Prüfverfahren. Im Falle von mehreren Aufnahmeeinheiten kann die Benutzereingabe mindestens eine Aufnahmeeinheit spezifizieren oder auch spezifizieren, dass alle eingesetzten Gasmessgeräte überprüft werden sollen.

Weiterhin betrifft die Erfindung ein Rückgabe-Verfahren, um ein Gasmessgerät in einen erfindungsgemäßen Ausgabeautomaten zurückzugeben. Vor der Durchführung des Rückgabe-Verfahrens ist folgende Situation hergestellt:
- In die Aufnahmeeinheit ist kein Gasmessgerät eingesetzt. Im Falle von mehreren Aufnahmeeinheiten ist in mindestens eine Aufnahmeeinheit kein Gasmessgerät eingesetzt.
- Der oder jeder Verschluss ist in der verschließenden Position.
- Die oder jede Arretiereinheit ist im arretierenden Zustand.

Ein Lesegerät des Ausgabeautomaten erfasst eine maschinenlesbare Kennung des Gasmessgeräts, das zurückgegeben werden soll. Beispielsweise liest das Lesegerät einen Datenspeicher, insbesondere einen NFC-Chip oder RFID-Chip, des Gasmessgerät aus oder erfasst einen Barcode oder einen QR-Code oder eine alphanumerische Kennung außen auf dem Gasmessgerät.

Der Schritt, dass die Kennung erfasst worden ist, löst folgenden Schritt aus: Die Steuereinheit prüft, ob die Aufnahmeeinheit das Gasmessgerät aufzunehmen vermag oder nicht. Das Gasmessgerät ist das Gerät, dessen Kennung erfasst worden ist. Beispielsweise ist mit der erfassten Kennung mindestens eine Abmessung und / oder eine Kennzeichnung einer elektrischen Schnittstelle verbunden, und die Steuereinheit prüft abhängig von der erfassten Abmessung und / oder der Kennzeichnung der Schnittstelle, ob die Aufnahmeeinheit geeignet ist oder nicht.

Im Falle von mehreren Aufnahmeeinheiten sucht die Steuereinheit nach einer Aufnahmeeinheit, in die aktuell kein Gasmessgerät eingesetzt worden ist und die das Gasmessgerät aufzunehmen vermag.

Die Feststellung, dass die oder eine Aufnahmeeinheit das Gasmessgerät aufzunehmen vermag, löst folgenden Schritt aus: Die Steuereinheit bewirkt, dass die Arretiereinheit für die geeignete Aufnahmeeinheit in den freigebenden Zustand überführt wird. Nunmehr lässt der Verschluss sich öffnen oder springt von allein auf, und das Gasmessgerät lässt sich in die Aufnahmeeinheit einsetzen. In einer Realisierungsform lässt sich das Gasmessgerät einsetzen, nachdem das Kontaktelement in die beabstandete Position bewegt worden ist.

Dieses Rückgabe-Verfahren reduziert das Risiko, dass das Gasmessgerät irgendwo in den Ausgabeautomaten eingelegt wird, und reduziert insbesondere das Risiko, dass das Gasmessgerät in eine nicht passende Aufnahmeeinheit eingesetzt wird. Das Rückgabe-Verfahren erfordert nicht notwendigerweise, dass ein Benutzer, der das Gasmessgerät zurückgeben will, sich selbst autorisieren muss. Außerdem erleichtert dieses Rückgabe-Verfahren es in vielen Fällen, dass der Ausgabeautomat das zurückgegebene Gasmessgerät überprüft.

In einer Ausgestaltung des Rückgabe-Verfahrens löst der Schritt, dass das Gasmessgerät in die oder eine Aufnahmeeinheit eingesetzt worden ist, folgende weiteren Schritte aus: Eine Gasprobe aus dem oben erwähnten Behälter mit Prüfgas wird zu dem gerade eingesetzten Gasmessgerät geleitet. Das Gasmessgerät misst die Zielgas-Konzentration, und die gemessene wird mit der vorgegebenen Zielgas-Konzentration verglichen. Dank dieser Ausgestaltung wird das Gasmessgerät unmittelbar nach der Rückgabe überprüft.

Im Falle von mehreren Aufnahmeeinheiten umfasst der Ausgabeautomat bevorzugt für jede Aufnahmeeinheit jeweils ein Ventil. Das Ventil für eine Aufnahmeeinheit wird geöffnet, wenn ein in diese Aufnahmeeinheit eingesetztes Gasmessgerät überprüft werden soll. Ansonsten ist das Ventil geschlossen.

Diese Realisierungsform ermöglicht es, ein bestimmtes Gasmessgerät gezielt zu überprüfen, beispielsweise ein gerade eingesetztes. Die Realisierungsform spart Prüfgas ein verglichen mit einer Ausgestaltung, bei der bei jeder Prüfung alle eingesetzten Gasmessgeräte überprüft werden.

Im Folgenden wird die Erfindung anhand eines Ausführungsbeispiels beschrieben. Hierbei zeigt
- Figur 1: in einer Draufsicht eine Prüfstation mit drei Aufnahmeeinheiten;
- Figur 2: in einer seitlichen Ansicht die Prüfstation von Figur 1;
- Figur 3: schematisch in einer Draufsicht einen erfindungsgemäßen Ausgabeautomaten;
- Figur 4: in einer Ansicht schräg von oben eine erste Realisierungsform eines erfindungsgemäßen Ausgabeautomaten;
- Figur 5: in einer Ansicht von oben einen Bereich des Ausgabeautomaten von Figur 4;
- Figur 6: in einer Ansicht schräg von oben einen Teil des Bereichs von Figur 5;
- Figur 7: in einer seitlichen Ansicht den Ausgabeautomaten von Figur 4;
- Figur 8: in einer Ansicht schräg von oben eine zweite Realisierungsform eines erfindungsgemäßen Ausgabeautomaten, wobei die Schutzklappe geöffnet ist;
- Figur 9: den Ausgabeautomaten von Figur 8 mit geschlossener Schutzklappe;
- Figur 10: in einer Ansicht schräg von hinten den Ausgabeautomaten von Figur 9;
- Figur 11: eine einzelne Aufnahmeeinheit des Ausgabeautomaten von Figur 8 bis Figur 10 mit geöffnetem Verschluss.

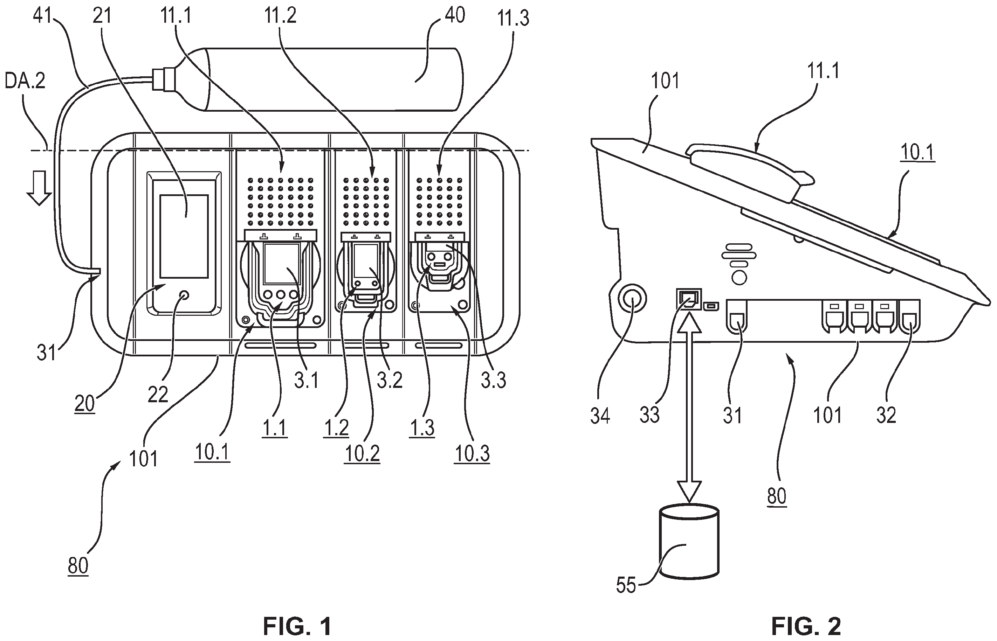

Figur 1 und Figur 2 zeigen eine Prüfstation 80 in Draufsicht bzw. in einer Seitenansicht. Die Prüfstation 80 umfasst drei Aufnahmeeinheiten 10.1, 10.2, 10.3, die von links nach rechts nebeneinander in einem Gehäuse 101 angeordnet sind.

Jede Aufnahmeeinheit 10.1, 10.2, 10.3 nimmt in einer Ausbuchtung jeweils ein Gasmessgerät 1.1, 1.2, 1.3 auf. Im gezeigten Beispiel sind die drei Aufnahmeeinheiten 10.1, 10.2, 10.3 unterschiedlich groß. Dies ist aber nicht zwingend erforderlich. Die Prüfstation 80 kann auch eine andere Anzahl von Aufnahmeeinheiten umfassen.

Jedes Gasmessgerät 1.1, 1.2, 1.3 umfasst eine eigene Spannungsversorgungseinheit und vermag das Ergebnis einer Messung auf einer Anzeigeeinheit 3.1, 3.2, 3.3 visuell darzustellen. In einer Ausgestaltung umfasst die Anzeigeeinheit 3.1, 3.2, 3.3 eine Hintergrundbeleuchtung.

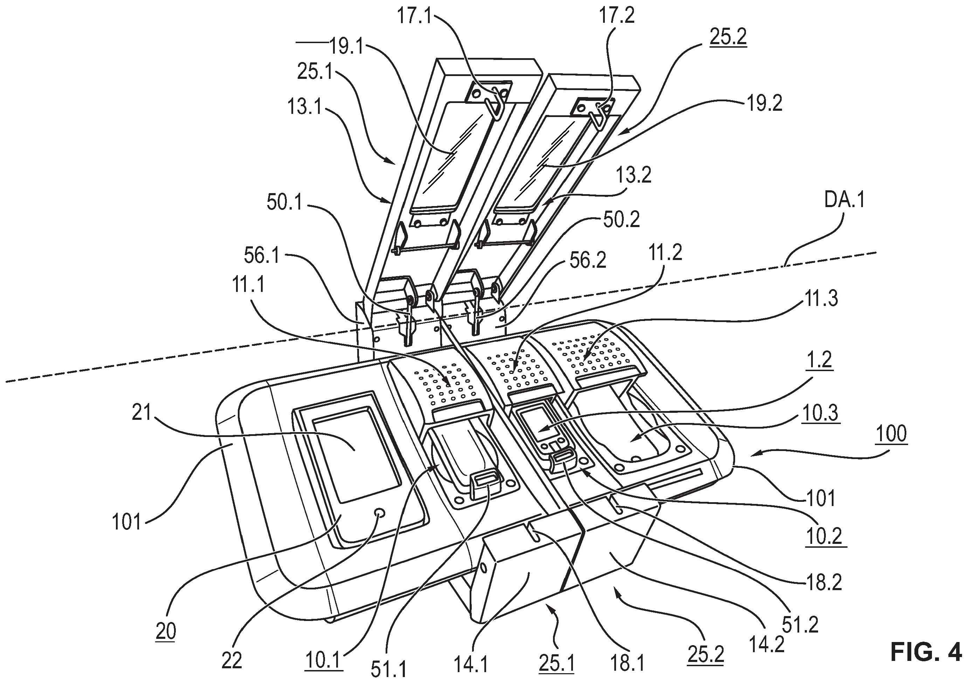

Jede Aufnahmeeinheit 10.1, 10.2, 10.3 umfasst jeweils eine elektrische Kontaktierung 51.1, 51.2, 51.3, vgl. Figur 4, Figur 5, Figur 7 und Figur 11. Solange ein Gasmessgerät 1.1, 1.2, 1.3 in eine Aufnahmeeinheit 10.1, 10.2, 10.3 eingesetzt ist, wird mittels der elektrischen Kontaktierung 51.1, 51.2, 51.3 oder durch induktives Laden die Spannungsversorgungseinheit dieses Gasmessgeräts 1.1, 1.2, 1.3 aufgeladen.

Bevorzugt umfasst jede Aufnahmeeinheit 10.1, 10.2, 10.3 jeweils einen Geräte-Sensor (nicht gezeigt). Dieser Geräte-Sensor vermag festzustellen, ob in die Aufnahmeeinheit 10.1, 10.2, 10.3 aktuell ein Gasmessgerät 1.1, 1.2, 1.3 eingesetzt ist oder nicht. Der Geräte-Sensor umfasst in einer Realisierungsform einen Kontaktschalter. Möglich ist auch, dass der Geräte-Sensor feststellt, ob ein Strom durch die elektrische Kontaktierung 51.1, 51.2, 51.3 fließt, was nur bei eingesetztem Gasmessgerät möglich ist.

Jede Aufnahmeeinheit 10.1, 10.2, 10.3 umfasst jeweils ein Kontaktelement 11.1, 11.2, 11.3. Durch dieses Kontaktelement 11.1, 11.2, 11.3 hindurch lässt sich ein Gasgemisch zu einem Gaseinlass 2.1, 2.2, 2.3 eines aufgenommenen Gasmessgeräts 1.1, 1.2, 1.3 leiten. Die drei Kontaktelemente 11.1, 11.2, 11.3 sind unabhängig voneinander relativ zum Gehäuse 101 um eine gemeinsame Drehachse DA.2 drehbar. In Figur 1 werden die drei Kontaktelemente 11.1, 11.2, 11.3 in einer nach unten gedrehten Position gezeigt, sie überdecken dann den jeweiligen Gaseinlass 2.1, 2.2, 2.3 eines aufgenommenen Gasmessgeräts 1.1, 1.2, 1.3. Wenn ein Kontaktelement 11.1, 11.2, 11.3 nach oben gedreht ist, lässt sich ein eingesetztes Gasmessgerät 1.1, 1.2, 1.3 aus der Aufnahmeeinheit 10.1, 10.2, 10.3 entnehmen, und dasselbe oder ein anderes Gasmessgerät lässt sich einsetzen. Die nach unten gedrehte Position ist die kontaktierende Position, die nach oben gedrehte Position die beabstandete Position.

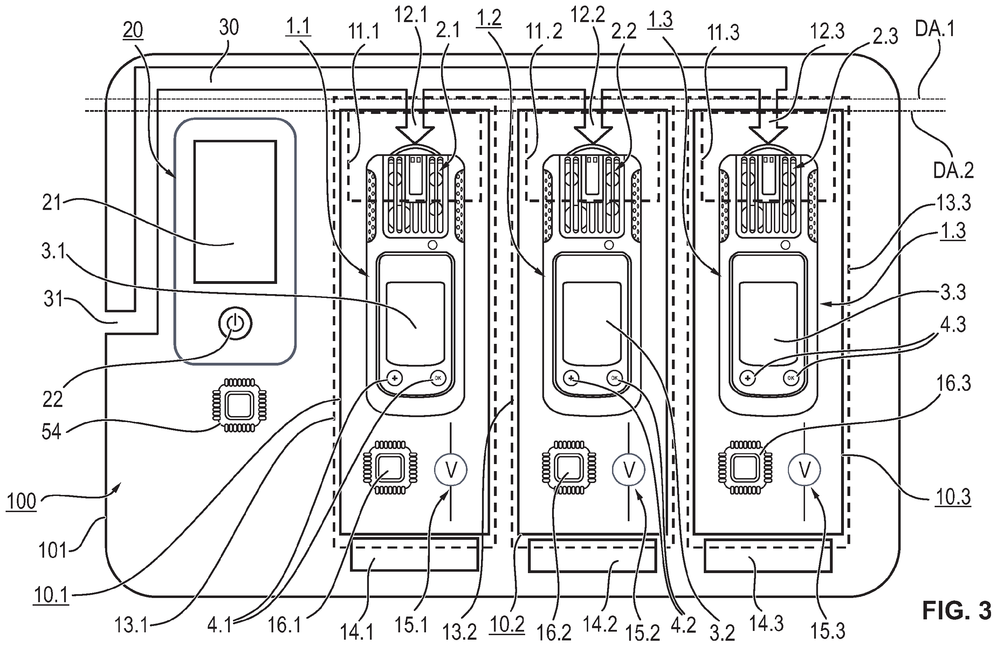

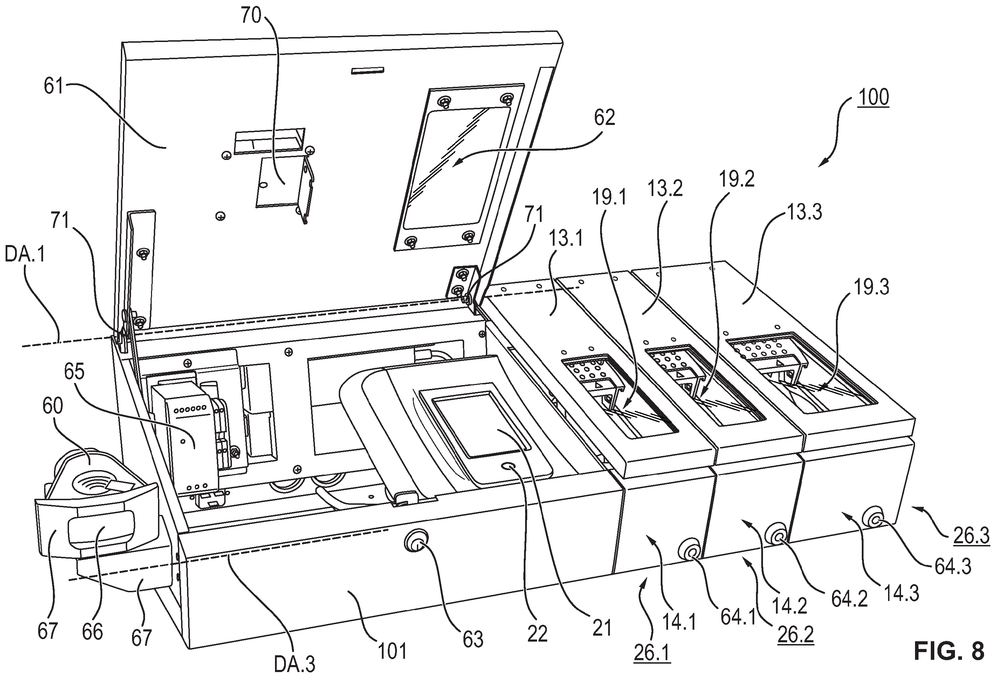

Figur 3 zeigt schematisch in einer Draufsicht einen erfindungsgemäßen Ausgabeautomaten 100, der die Prüfstation 80 von Figur 1 und Figur 2 umfasst. Figur 4 bis Figur 7 zeigen eine erste Realisierungsform, bei der die Prüfstation 80 von Figur 1 und Figur 2 um mindestens ein Verschluss-Bauteil ergänzt und dadurch ein erfindungsgemäßer Ausgabeautomat 100 bereitgestellt wird. Im gezeigten Beispiel ist um die beiden Aufnahmeeinheiten 10.1 und 10.2 jeweils ein Verschluss-Bauteil 25.1, 25.2 angebracht, während um die Aufnahmeeinheit 10.3 kein Verschluss-Bauteil angebracht ist. Figur 8 bis Figur 11 zeigen eine zweite Realisierungsform, bei welcher der Ausgabeautomat 100 drei Aufnahme-Module 26.1, 26.2, 26.3 umfasst. Gleiche Bezugszeichen haben die gleichen Bedeutungen.

Figur 1 zeigt einen Behälter 40 in Form einer Flasche. Der Behälter 40 nimmt ein Prüfgas auf, und zwar bevorzugt unter Überdruck. Dieses Prüfgas umfasst mindestens ein Zielgas mit einer bekannten Konzentration. Eine Leitung 41 leitet das Prüfgas aus dem Behälter 40 zu einem Gaseinlass 31 im Gehäuse 101. Die Leitung 41 fungiert als die externe Fluidführungseinheit - nämlich extern vom Ausgabeautomaten 100. Eine Gasleitung 30 verbindet den Gaseinlass 31 mit Gaszuführelementen 12.1, 12.2, 12.3 in den Kontaktelementen 11.1, 11.2, 11.3, vgl. Figur 3. Die Gasleitung 30 und die Kontaktelemente 11.1, 11.2, 11.3 mit den Gaszuführelementen 12.1, 12.2, 12.3 gehören zu der Fluidführungs-Anordnung des Ausführungsbeispiels. Bevorzugt befindet sich im Inneren eines Kontaktelements 11.1, 11.2, 11.3 ein ansteuerbares Ventil oder sonstiges pneumatisches Steuerelement. Abhängig von der Ansteuerung gibt das Ventil das Gaszuführelement 12.1, 12.2, 12.3 frei oder versperrt es. Bevorzugt wird mittels eines Volumenflusssensors oder eines Drucksensors (nicht gezeigt) überprüft, ob die Fluidführungs-Anordnung ein Leck aufweist.

Solange der Behälter 40 mittels der Leitung 41 mit dem Gaseinlass 31 verbunden ist, wird das Prüfgas aus dem Behälter 40 durch die Leitungen 41 und 30 hindurch zu den Gaszuführelementen 12.1, 12.2, 12.3 geleitet. In einer Ausgestaltung ist der Behälter 40 während eines Einsatzes der Prüfstation 80 dauerhaft mit der Prüfstation 80 und damit mit dem Ausgabeautomaten 100 verbunden. Optional vermag ein nicht gezeigter Deckel den Behälter 40 zu verschließen.

Eine Anzeige- und Bedieneinheit 20 umfasst einen Bildschirm 21, bevorzugt einen berührungssensitiven Bildschirm (Touch Screen) sowie einen Einschaltknopf 22, vgl. Figur 1. Ein Benutzer kann mithilfe des Bildschirms 21 Benutzereingaben tätigen. Bei der ersten Realisierungsform sind der Bildschirm 21 und der Einschaltknopf 22 für jeden Benutzer zugänglich, vgl. Figur 3 bis Figur 5. Der Ausgabeautomat 100 gemäß der zweiten Realisierungsform umfasst eine Schutzklappe 61, die mittels eines Scharniers 71 am Gehäuse 101 befestigt ist und sich relativ zum Gehäuse 101 um die gleiche Drehachse DA.1 drehen lässt wie die Verschlüsse 13.1, 13.2, 13.3. Die herabgeklappte Schutzklappe 61 lässt sich mithilfe eines mechanischen Schlosses 63 im Gehäuse 101 und eines passenden Schlüssels verschließen und bevorzugt auch wieder öffnen, ähnlich einer Geldkassette. Anstelle des oder zusätzlich zu dem mechanischen Schloss 63 kann auch ein elektronisches Schloss (nicht gezeigt) vorhanden sein.

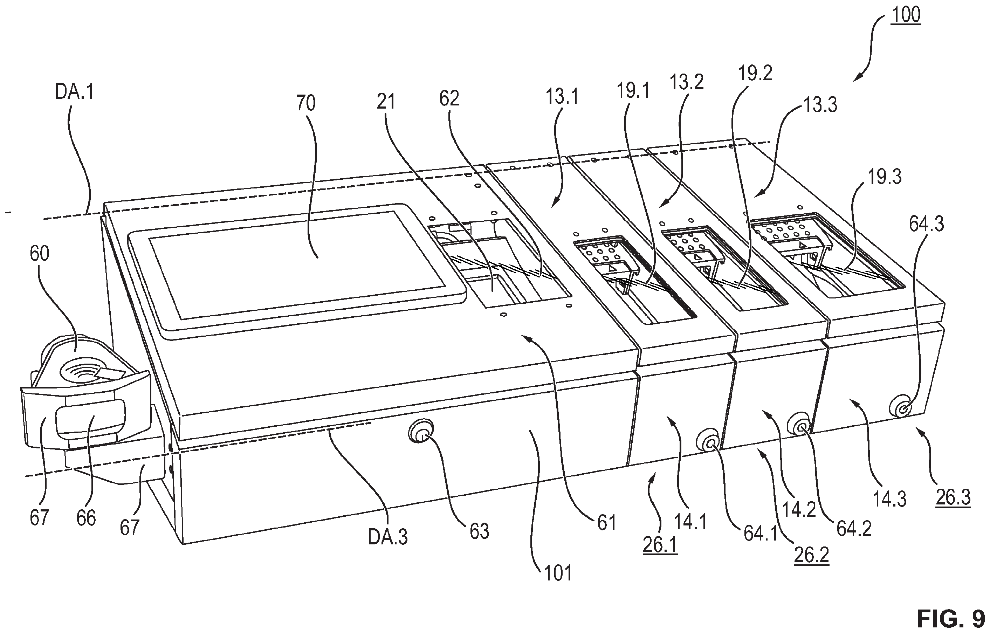

In die Schutzklappe 61 ist ein berührungssensitiver Bildschirm 70 eingelassen, vgl. Figur 9. Ein Benutzer tätigt bei verschlossener Schutzklappe 61 Benutzereingaben auf dem Bildschirm 70. Der Bildschirm 21 ist bei geschlossener Schutzklappe 61 durch ein Fenster 62 hindurch sichtbar, kann nur bei geöffneter Schutzklappe 61 betätigt werden und ist dafür bestimmt, von einem Wartungstechniker verwendet zu werden. Entsprechend werden Ausgaben an einen Benutzer auf dem Bildschirm 70 ausgegeben, beispielsweise die Information, dass ein Gasmessgerät mit erforderlichen oder gewünschten Eigenschaften aktuell nicht verfügbar ist. Ausgaben an einen Wartungstechniker, beispielsweise Informationen über den aktuellen Zustand eines eingesetzten Gasmessgeräts 1.1, 1.2, 1.3 oder des Ausgabeautomaten 100, werden hingegen auf dem Bildschirm 21 ausgegeben.

Solange ein Gasmessgerät 1.1, 1.2, 1.3 in eine Aufnahmeeinheit 10.1, 10.2, 10.3 eingesetzt und der Behälter 40 angeschlossen ist, wird eine Gasprobe des Prüfgases durch den Gaseinlass 2.1, 2.2, 2.3 hindurch in die Messkammer des Gasmessgeräts 1.1, 1.2, 1.3 geleitet. Der Sensor des Gasmessgeräts 1.1, 1.2, 1.3 misst die Zielgas-Konzentration in der Gasprobe in der Messkammer. Auf der Anzeige- und Bedieneinheit 20 und / oder auf der eigenen Anzeigeeinheit 3.1, 3.2, 3.3 wird für jedes eingesetzte Gasmessgerät 1.1, 1.2, 1.3 jeweils das Ergebnis der Messung angezeigt, bei drei eingesetzten Gasmessgeräten 1.1, 1.2, 1.3 also drei Messungen. Optional wird zusätzlich die vorgegebene Konzentration des Zielgases in der Gasprobe angezeigt, wobei diese Gasprobe vom Prüfgas aus dem Behälter 40 stammt. Natürlich ist es möglich, dass nacheinander mehrere Behälter mit unterschiedlichen Prüfgasen verwendet werden.

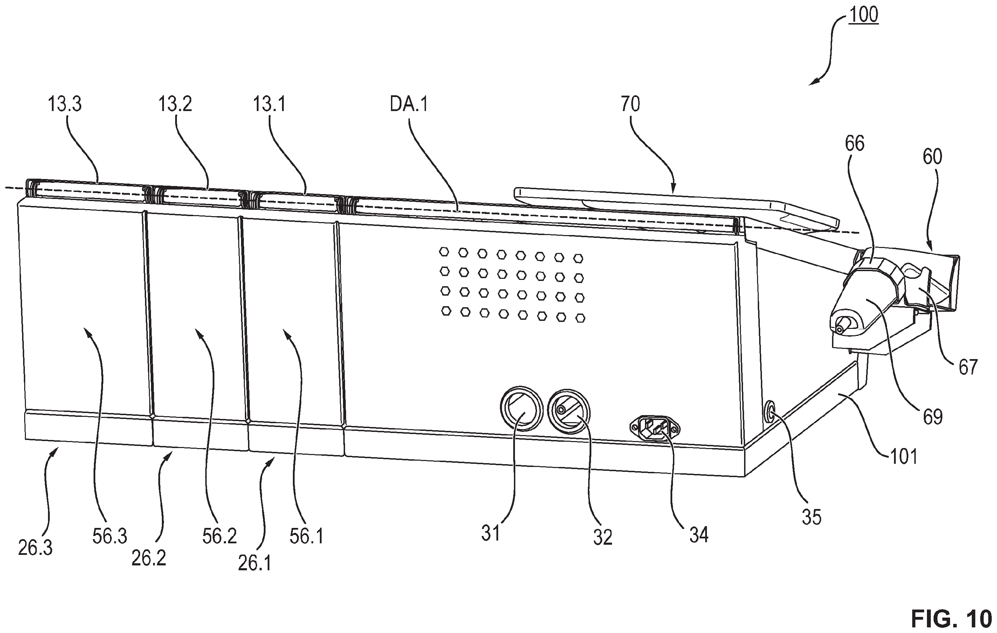

In der Seitenansicht von Figur 2 sowie in Figur 10 werden der Gaseinlass 31, ein weiterer Gaseinlass 32, eine Steckdose 35 für ein Scanner-Kabel und eine Steckdose 34 gezeigt. Eine nicht gezeigte Gasleitung verbindet den weiteren Gaseinlass 32 mit der Gasleitung 30 oder mit den Gaszuführelementen 12.1, 12.2, 12.3. In die Steckdose 34 lässt sich ein Stecker einstecken, um die Prüfstation 80 und den Ausgabeautomaten 100 mit einem stationären Spannungsversorgungsnetz zu verbinden und dadurch mit elektrischer Energie zu versorgen. Optional umfassen die Prüfstation 80 und der Ausgabeautomat 100 eine eigene Spannungsversorgungseinheit, bevorzugt eine aufladbare Spannungsversorgungseinheit.

Im Ausführungsbeispiel sind die Prüfstation 80 und der Ausgabeautomat 100 über ein Ethernet-Kabel mit einer schematisch gezeigten Datenbank 55 verbunden. Das Ethernet-Kabel ist in einen Anschluss 33 eingesteckt, vgl. Figur 2. In der Datenbank 55 ist für jedes Gasmessgerät einer Menge von Gasmessgeräten, darunter für die Gasmessgeräte 1.1, 1.2, 1.3, jeweils ein Datensatz angelegt. Der Datensatz für ein Gasmessgerät 1.1, 1.2, 1.3 umfasst eine eindeutige Kennung, eine Information über den Typ und eine Information über den aktuellen Status des Gasmessgeräts 1.1, 1.2, 1.3. Zur Information über den Typ gehört eine Kennzeichnung, welches Zielgas oder welche Zielgase das Gasmessgerät 1.1, 1.2, 1.3 zu messen vermag. Zur Information über den aktuellen Status gehört eine Kennzeichnung, ob das Gasmessgerät 1.1, 1.2, 1.3 aktuell in eine Aufnahmeeinheit 10.1, 10.2, 10.3 des Ausgabeautomaten 100 eingesetzt ist, bevorzugt eine Information, in welche Aufnahmeeinheit, oder ob das Gasmessgerät 1.1, 1.2, 1.3 aktuell verwendet wird oder sich in einer Reparatur befindet. Außerdem gehört bevorzugt zur Status-Information eine Kennzeichnung, ob das Gasmessgerät 1.1, 1.2, 1.3 bei der letzten Prüfung als intakt oder defekt eingestuft worden ist.

Der Ausgabeautomat 100 umfasst ein signalverarbeitendes Steuergerät 54, vgl. Figur 3, sowie für jede Aufnahmeeinheit 10.1, 10.2, 10.3 jeweils
- einen Verschluss 13.1, 13.2, 13.3,
- eine Arretiereinheit 14.1, 14.2, 14.3 für den Verschluss 13.1, 13.2, 13.3,
- einen Spannungssensor 15.1, 15.2, 15.3 und
- eine signalverarbeitende Auswerteeinheit 16.1, 16.2, 16.3.

Alternativ führt eine Steuereinheit 65 sowohl die Funktionen des Steuergeräts 54 als auch die Funktionen der Auswerteeinheiten 16.1, 16.2, 16.3 durch, vgl. Figur 8. Nachfolgend wird die Bezeichnung "die Steuereinheit 16.1, 16.2, 16.3, 54, 65" verwendet.

Der Spannungssensor 15.1, 15.2, 15.3 einer Aufnahmeeinheit 10.1, 10.2, 10.3 misst den aktuellen Ladezustand der Spannungsversorgungseinheit eines eingesetzten Gasmessgeräts 1.1, 1.2, 1.3.

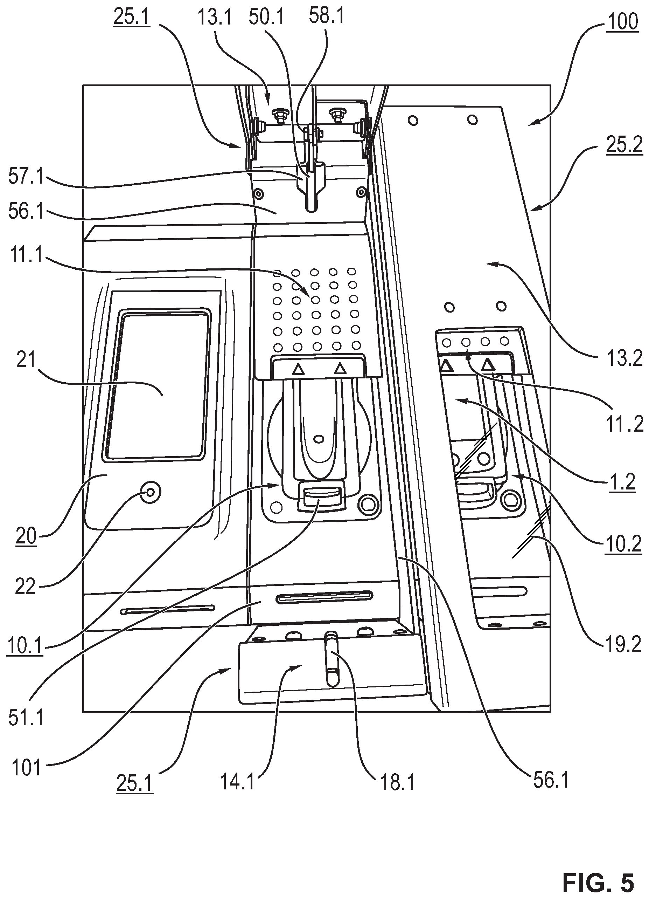

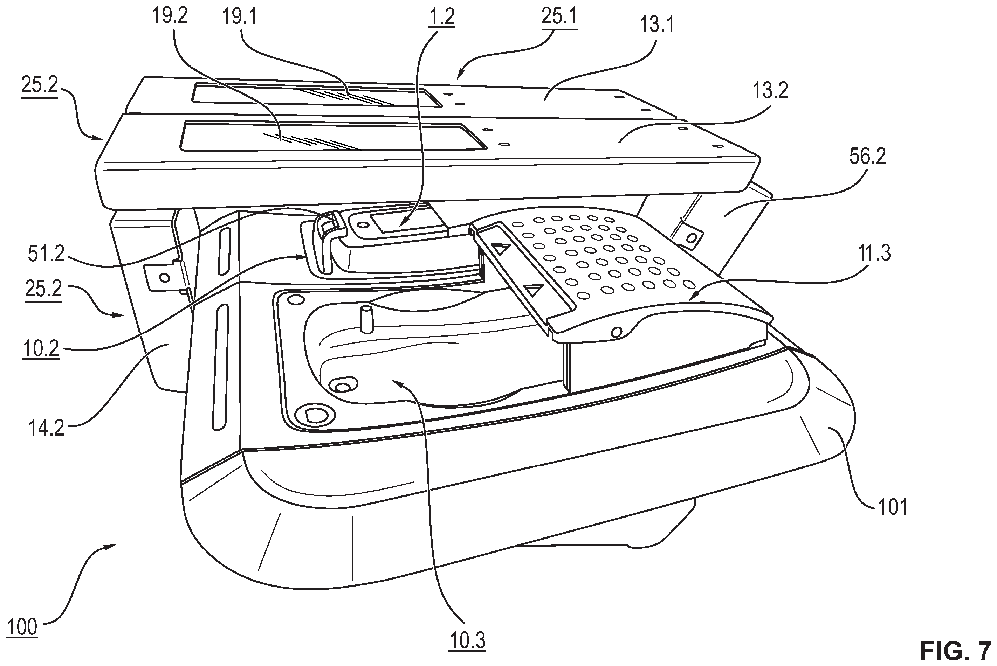

Jeder Verschluss 13.1, 13.2, 13.3 ist relativ zum Gehäuse 101 um eine Drehachse DA.1 drehbar und ist jeweils einer Aufnahmeeinheit 10.1, 10.2, 10.3 zugeordnet. Die Drehachse DA.1 ist parallel zur Drehachse DA.2, um welche die Kontaktelemente 11.1, 11.2, 11.3 drehbar sind. Die beiden Drehachsen DA.1 und DA.2 liegen in der Zeichenebene von Figur 1 und Figur 3 und stehen senkrecht auf der Zeichenebene von Figur 2. In der schematischen Darstellung von Figur 3 sowie in Figur 8 bis Figur 10 sind die Verschlüsse 13.1, 13.2, 13.3 abgesenkt und daher in der verschließenden Position. Das Kontaktelement 11.1, 11.2, 11.3 einer Aufnahmeeinheit 10.1, 10.2, 10.3 befindet sich bei abgesenktem Verschluss 13.1, 13.2, 13.3 zwischen dem Verschluss 13.1, 13.2, 13.3 und einem in die Aufnahmeeinheit 10.1, 10.2, 10.3 eingesetzten Gasmessgerät 1.1, 1.2, 1.3 oder auch der leeren Ausbuchtung. In der Darstellung von Figur 4 sind die beiden Verschlüsse 13.1 und 13.2 nach oben gedreht (geklappt), und der Verschluss 13.3 wird nicht gezeigt. Alle drei Kontaktelemente 11.1, 11.2, 11.3 sind nach unten gedreht. In die Aufnahmeeinheit 10.2 ist ein Gasmessgerät 1.2 eingesetzt, während in die übrigen beiden Aufnahmeeinheiten 10.1 und 10.3 kein Gasmessgerät eingesetzt ist. In Figur 5 sind der Verschluss 13.1 nach oben und der Verschluss 13.2 nach unten gedreht. In der Seitenansicht von Figur 7 sind die beiden Verschlüsse 13.1 und 13.2 nach unten gedreht.

Jeder Verschluss 13.1, 13.2, 13.3 umfasst jeweils
- einen rechteckigen Rahmen, der im Ausführungsbeispiel aus Metall ist,
- ein Fenster 19.1, 19.2, 19.3 und
- einen Vorsprung 17.1, 17.2, 17.3, der zum Gehäuse 101 hin zeigt.

Falls der Verschluss 13.1, 13.2, 13.3 eine Aufnahmeeinheit 10.1, 10.2, 10.3 verschließt, so befindet sich der Verschluss 13.1, 13.2, 13.3 mit dem Fenster 19.1, 19.2, 19.3 oberhalb der Aufnahmeeinheit 10.1, 10.2, 10.3, so dass ein aufgenommenes Gasmessgerät 1.1, 1.2, 1.3 durch das Fenster 19.1, 19.2, 19.3 hindurch sichtbar ist.

Jeder Verschluss 13.1, 13.2, 13.3 ist an jeweils einer Halterung 56.1, 56.2, 56.3 drehbar befestigt. Diese Halterung 56.1, 56.2, 56.3 ist mechanisch mit dem Gehäuse 101 verbunden und umfasst eine Längswand. Jede Arretiereinheit 14.1, 14.2, 14.3 ist ebenfalls mechanisch mit dem Gehäuse 101 verbunden und kann sich bevorzugt nicht relativ zum Gehäuse 101 bewegen. Die Aufnahmeeinheit 10.1, 10.2, 10.3 befindet sich zwischen der zugeordneten Arretiereinheit 14.1, 14.2, 14.3 und der Halterung 56.1, 56.2, 56.3 für den zugeordneten Verschluss 13.1, 13.2, 13.3.

Jede Arretiereinheit 14.1, 14.2, 14.3 ist jeweils einem Verschluss 13.1, 13.2, 13.3 zugeordnet und lässt sich in einen arretierenden und in einen freigebenden Zustand verbringen (überführen), und zwar unabhängig von jeder anderen Arretiereinheit. Im arretierenden Zustand arretiert die Arretiereinheit 14.1, 14.2, 14.3 den zugeordneten nach unten gedrehten Verschluss 13.1, 13.2, 13.3. Solange die Arretiereinheit 14.1, 14.2, 14.3 im arretierenden Zustand ist, ist es nicht möglich, den zugeordneten Verschluss 13.1, 13.2, 13.3 zu öffnen. Dann lässt sich weder ein Gasmessgerät 1.1, 1.2, 1.3 aus der Aufnahmeeinheit 10.1, 10.2, 10.3 entnehmen, noch lässt sich ein Gasmessgerät einsetzen. Wenn die Arretiereinheit 14.1, 14.2, 14.3 im freigebenden Zustand ist, lässt sich der zugeordnete Verschluss 13.1, 13.2, 13.3 relativ zum Gehäuse 101 um die Drehachse DA.1 drehen und daher in die geöffnete Position überführen.

In jede Arretiereinheit 14.1, 14.2, 14.3 ist jeweils eine Aussparung 18.1, 18.2, 18.3 eingelassen. Falls der zugeordnete Verschluss 13.1, 13.2, 13.3 nach unten gedreht ist, so greift der Vorsprung 17.1, 17.2, 17.3 an diesem Verschluss 13.1, 13.2, 13.3 in die Aussparung 18.1, 18.2, 18.3 ein. Im Inneren der Arretiereinheit 14.1, 14.2, 14.3 befindet sich ein beweglich gelagerter Arretierkörper (nicht gezeigt). Wenn die Arretiereinheit 14.1, 14.2, 14.3 im arretierenden Zustand ist, so greift dieser Arretierkörper in den Vorsprung 17.1, 17.2, 17.3 ein und verhindert, dass der Verschluss 13.1, 13.2, 13.3 nach oben gedreht werden kann. Der Arretierkörper lässt sich aus dem Vorsprung 17.1, 17.2, 17.3 herausziehen. Wenn die Arretiereinheit 14.1, 14.2, 14.3 im freigebenden Zustand ist, greift der Arretierkörper nicht in den Vorsprung 17.1, 17.2, 17.3 ein.

In jede Arretiereinheit 14.1, 14.2, 14.3 ist weiterhin ein ansteuerbares und ebenfalls nicht gezeigtes Stellglied eingesetzt, insbesondere ein elektrisches oder pneumatisches oder hydraulisches Stellglied. Dieses Stellglied vermag den Arretierkörper relativ zur Aussparung 18.1, 18.2, 18.3 zu bewegen.

In einer Ausgestaltung vermag das Stellglied den Arretierkörper hin und her zu bewegen und dadurch sowohl zu bewirken, dass der Arretierkörper in den Vorsprung 17.1, 17.2, 17.3 eingreift, als auch zu bewirken, dass der Arretierkörper aus dem Vorsprung 17.1, 17.2, 17.3 herausgezogen wird.

In einer anderen Ausgestaltung ist in jede Arretiereinheit 14.1, 14.2, 14.3 weiterhin ein mechanisches Rückstellelement eingesetzt, beispielsweise eine Feder, wobei das Rückstellelement eine rückstellende Kraft ausübt. Das Stellglied vermag den Arretierkörper gegen die rückstellende Kraft in die eine Richtung zu bewegen. Falls das Stellglied nicht aktiv ist, so bewegt die rückstellende Kraft den Arretierkörper in die entgegengesetzte Richtung und hält ihn in einer Position.

In einer Realisierungsform dieser anderen Ausgestaltung bewirkt die rückstellende Kraft, dass der Arretierkörper aus dem Vorsprung 17.1, 17.2, 17.3 herausgezogen wird. Diese Realisierungsform hat folgende Wirkung: Falls das Stellglied ausfällt oder nicht mehr mit elektrischer oder hydraulischer oder pneumatischer Energie versorgt wird, so überführt die rückstellende Kraft des mechanischen Rückstellelements die Arretiereinheit 14.1, 14.2, 14.3 in den freigebenden Zustand. In einem Notfall lässt sich dann das Gasmessgerät 1.1, 1.2, 1.3 aus der Aufnahmeeinheit 10.1, 10.2, 10.3 entnehmen.

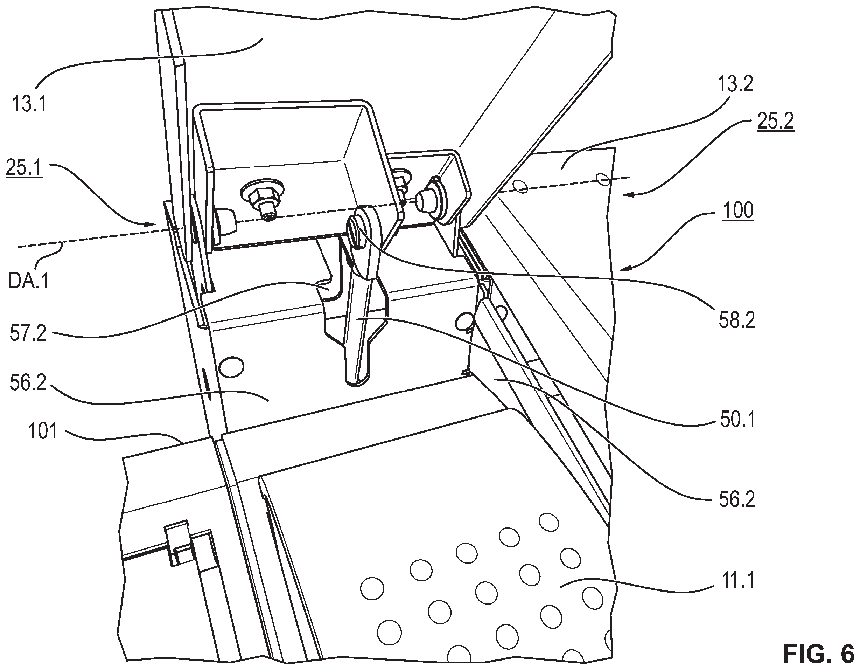

Im Ausführungsbeispiel ist in jede Halterung 56.1, 56.2, 56.3 jeweils ein Stab 50.1, 50.2, 50.3 drehbar eingesetzt. Dieser Stab 50.1, 50.2, 50.3 fungiert als das Öffnungselement. Dank einer Aussparung 57.1, 57.2, 57.3 kann der Stab 50.1, 50.2, 50.3 sich relativ zur Halterung 56.1, 56.2, 56.3 drehen, und zwar in eine Richtung senkrecht zur Drehachse DA.1, vgl. Figur 6. Eine nicht gezeigte Feder ist bestrebt, den Stab 50.1, 50.2, 50.3 in eine aufrechte Position zu drehen und in dieser zu halten. Der Stab 50.1, 50.2, 50.3 bewirkt oder unterstützt insbesondere die nachfolgend beschriebenen Schritte.

Wie bereits dargelegt, verhindert die Arretiereinheit 14.1, 14.2, 14.3 im arretierenden Zustand, dass der nach unten gedrehte Verschluss 13.1, 13.2, 13.3 sich öffnen lässt. Der nach unten gedrehte Verschluss 13.1, 13.2, 13.3 hält den Stab 50.1, 50.2, 50.3 gegen die Federkraft in einer herabgedrückten Position. Sobald die Arretiereinheit 14.1, 14.2, 14.3 in den freigebenden Zustand überführt wird, der Verschluss 13.1, 13.2, 13.3 also dearretiert wird, dreht die Feder den Stab 50.1, 50.2, 50.3 nach oben, also vom Kontaktelement 11.1, 11.2, 11.3 weg. Dadurch wird der dearretierte Verschluss 13.1, 13.2, 13.3 von allein geöffnet, springt also auf. Ein Benutzer bemerkt dadurch, dass er nunmehr das Gasmessgerät aus der Aufnahmeeinheit 10.1, 10.2, 10.3 entnehmen kann.

Bevorzugt befindet sich am freien Ende des federnd gelagerten Stabs 50.1, 50.2, 50.3 eine drehbar gelagerte Walze 58.1, 58.2, 58.3. Während der Verschluss 13.1, 13.2, 13.3 nach oben gedreht wird, rollt die Walze 58.1, 58.2, 58.3 am Stab 50.1, 50.2, 50.3 über die nach unten zeigende Oberfläche des Verschlusses 13.1, 13.2, 13.3. Dadurch verhindert die Walze 58.1, 58.2, 58.3, dass der Stab 50.1, 50.2, 50.3 über den Verschluss 13.1, 13.2, 13.3 kratzt oder gar sich verhakt.

In der aufrechten Position verhindert der Stab 50.1, 50.2, 50.3, dass der nach oben gedrehte Verschluss 13.1, 13.2, 13.3 aufgrund der Schwerkraft von allein nach unten gedreht wird, also zufällt. Der Verschluss 13.1, 13.2, 13.3 lässt sich gegen die Kraft der Feder verschließen.

Ein Benutzer gibt ein Gasmessgerät 1.1, 1.2, 1.3 zurück, indem der Benutzer das Gasmessgerät 1.1, 1.2, 1.3 in eine passende Aufnahmeeinheit 10.1, 10.2, 10.3 einlegt. Anschließend sind folgende Schritte erforderlich: Das Kontaktelement 11.1, 11.2, 11.3 wird nach unten gedreht, und der optionale Rastverschluss hält das Kontaktelement 11.1, 11.2, 11.3 in der nach unten gedrehten Position. Der Verschluss 13.1, 13.2, 13.3 wird nach unten gedreht, bis der Vorsprung 17.1, 17.2, 17.3 in die Aussparung 18.1, 18.2, 18.3 eingreift. Die Arretiereinheit 14.1, 14.2, 14.3 arretiert den Verschluss 13.1, 13.2, 13.3.

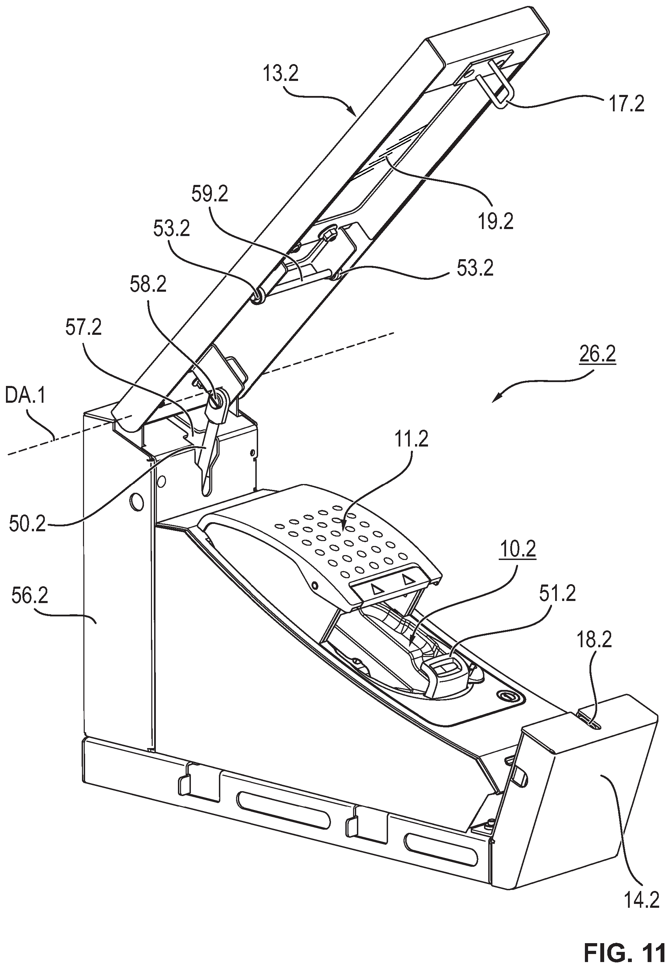

Nachfolgend wird eine Realisierungsform beschrieben, dank der es nicht erforderlich ist, zunächst das Kontaktelement 11.1, 11.2, 11.3 und anschließend den Verschluss 13.1, 13.2, 13.3 nach unten zu drehen. Vielmehr ist an der zum Kontaktelement 11.1, 11.2, 11.3 zeigenden Oberfläche des Verschlusses 13.1, 13.2, 13.3 ein Abstandselement 59.1, 59.2, 59.3 befestigt, vgl. Figur 11. Am freien Ende des Abstandselements 59.1, 59.2, 59.3 sind jeweils zwei Walzen 53.1, 53.2, 53.3 befestigt. Das Abstandselement 59.1, 59.2, 59.3 überträgt eine Drehung des Verschlusses 13.1, 13.2, 13.3 in Richtung der Aufnahmeeinheit 10.1, 10.2, 10.3 dergestalt auf das Kontaktelement 11.1, 11.2, 11.3, dass auch das Kontaktelement 11.1, 11.2, 11.3 auf die Aufnahmeeinheit 10.1, 10.2, 10.3 zu bewegt wird. Während der Verschluss 13.1, 13.2, 13.3 auf das Kontaktelement 11.1, 11.2, 11.3 zu bewegt wird, rollen die Walzen 53.1, 53.2, 53.3 über die nach oben zeigende Oberfläche des jeweiligen Kontaktelements 11.1, 11.2, 11.3.

Bevorzugt umfasst jede Aufnahmeeinheit 10.1, 10.2, 10.3 jeweils einen Verschlusssensor (nicht gezeigt), der bevorzugt einen Kontaktschalter umfasst. Der Verschlusssensor ermittelt, ob der zugeordnete Verschluss 13.1, 13.2, 13.3 aktuell nach oben oder nach unten gedreht ist. Der Verschluss 13.1, 13.2, 13.3 lässt sich im Ausführungsbeispiel nur dann arretieren, wenn er vollständig nach unten gedreht ist.

Bei der zweiten Realisierungsform ist der Arretiereinheit 14.1, 14.2, 14.3 ein mechanisches Schloss 64.1, 64.2, 64.3 zugeordnet. Das mechanische Schloss 64.1, 64.2, 64.3 lässt sich mithilfe eines passenden Schlüssels (nicht gezeigt) öffnen, auch wenn der Ausgabeautomat 100 nicht mit elektrischer Energie versorgt wird oder defekt ist.

Das mechanische Schloss 64.1, 64.2, 64.3 lässt sich mithilfe des passenden Schlüssels in einen verschließenden Zustand und in einen freigegebenen Zustand überführen. Bevorzugt ist das mechanische Schloss 64.1, 64.2, 64.3 in einen Verschluss 13.1, 13.2, 13.3 eingebettet und wie folgt mit der zugeordneten Arretiereinheit 14.1, 14.2, 14.3 verbunden: Wenn das mechanische Schloss 64.1, 64.2, 64.3 mithilfe des Schlüssels in den freigebenden Zustand überführt wird, wird auch die zugeordnete Arretiereinheit 14.1, 14.2, 14.3 in den freigebenden Zustand überführt, und der Verschluss 13.1, 13.2, 13.3 lässt sich öffnen oder springt auf. In einer Realisierungsform bewirkt umgekehrt der Vorgang, dass die Arretiereinheit 14.1, 14.2, 14.3 in die arretierende Position überführt wird, dass auch das Schloss 64.1, 64.2, 64.3 in den verschließenden Zustand überführt wird.

Anstelle eines Schlosses kann auch ein Betätigungselement eingelassen sein, wobei das Betätigungselement sich von außen betätigen lässt, um den Verschluss 13.1, 13.2, 13.3 zu öffnen.

Bei der ersten Realisierungsform (Figur 4 bis Figur 7) umfasst das Verschluss-Bauteil 25.1, 25.2 den Verschluss 13.1, 13.2 und die Arretiereinheit 14.1, 14.2. Bei der zweiten Realisierungsform (Figur 8 bis Figur 11) umfasst das Aufnahme-Modul 26.1, 26.2, 26.3 die Aufnahmeeinheit 10.1, 10.2, 10.3, den Verschluss 13.1, 13.2, 13.3, die Arretiereinheit 14.1, 14.2, 14.3, die Halterung 56.1, 56.2, 56.3 und das Schloss 64.1, 64.2, 64.3.

Die Steuereinheit 16.1, 16.2, 16.3, 54, 65 empfängt und verarbeitet die folgenden Signale und sonstigen Informationen über ein Gasmessgerät 1.1, 1.2, 1.3, das aktuell in die Aufnahmeeinheit 10.1, 10.2, 10.3 eingesetzt ist:
- In einer ersten Realisierungsform empfängt die Steuereinheit 16.1, 16.2, 16.3, 54, 65 über die elektrische Kontaktierung 51.1, 51.2, 51.3 eine rechnerverfügbare Beschreibung, die in einem Datenspeicher des eingesetzten Gasmessgeräts 1.1, 1.2, 1.3 abgespeichert ist. Diese Beschreibung umfasst eine eindeutige Kennung des Gasmessgeräts 1.1, 1.2, 1.3 und eine Information darüber, welches Zielgas oder welche Zielgase dieses Gasmessgerät 1.1, 1.2, 1.3 zu messen vermag, optional weitere Informationen über das Gasmessgerät 1.1, 1.2, 1.3. In einer Ausgestaltung umfasst diese Beschreibung außerdem eine Kennzeichnung, welche Qualifikation ein Benutzer des Gasmessgeräts 1.1, 1.2, 1.3 aufweisen muss, um das Gasmessgerät 1.1, 1.2, 1.3 benutzen zu dürfen. Optional umfasst diese Beschreibung außerdem eine Spezifikation, bei welchen Umgebungsbedingungen dieses Gasmessgerät 1.1, 1.2, 1.3 eingesetzt werden darf.
- In einer zweiten Realisierungsform ermittelt die Steuereinheit 16.1, 16.2, 16.3, 54, 65 diese Information durch eine Lesezugriff auf die Datenbank 55, wofür das Ethernet-Kabel verwendet wird, welches in den Anschluss 33 eingesteckt ist.
- Diese beiden Realisierungsformen lassen sich miteinander kombinieren, insbesondere um Redundanz zu erzeugen.
- Die Steuereinheit 16.1, 16.2, 16.3, 54, 65 empfängt und verarbeitet ein Signal des Spannungssensors 15.1, 15.2, 15.3 und ermittelt den aktuellen Ladezustand der Spannungsversorgungseinheit des eingesetzten

Gasmessgeräts 1.1, 1.2, 1.3.
- Die Steuereinheit 16.1, 16.2, 16.3, 54, 65 empfängt und verarbeitet ein Signal des Sensors des eingesetzten Gasmessgeräts 1.1, 1.2, 1.3. Dieses Signal umfasst eine Information über die Zielgas-Konzentration, die der Sensor des eingesetzten Gasmessgeräts 1.1, 1.2, 1.3 gemessen hat. Die gemessene Zielgas-Konzentration bezieht sich auf die Konzentration eines Zielgases in einer Gasprobe, wobei die Gasprobe von einem Prüfgas stammt, welches aus dem Behälter 40 in die Messkammer des Gasmessgeräts 1.1, 1.2, 1.3 geleitet worden ist, vgl. Figur 1.
- Die Steuereinheit 16.1, 16.2, 16.3, 54, 65 empfängt und verarbeitet ein Signal, welches eine Information über die vorgegebene und bekannte Zielgas-Konzentration des Prüfgases im Behälter 40 umfasst.

Im Ausführungsbeispiel ermittelt die Steuereinheit 16.1, 16.2, 16.3, 54, 65 den aktuellen Status des eingesetzten Gasmessgeräts 1.1, 1.2, 1.3 wie folgt:
- Die Steuereinheit 16.1, 16.2, 16.3, 54, 65 ermittelt den aktuellen Ladezustand der Spannungsversorgungseinheit des Gasmessgeräts 1.1, 1.2, 1.3. Falls der ermittelte Ladezustand kleiner ist als eine vorgegebene untere Schranke, so lässt sich das Gasmessgerät 1.1, 1.2, 1.3 aktuell nicht verwenden. In der Regel wird die Spannungsversorgungseinheit aufgeladen, während das Gasmessgerät 1.1, 1.2, 1.3 eingesetzt ist.
- Optional sagt die Steuereinheit 16.1, 16.2, 16.3, 54, 65 eine Zeitspanne vorher. Nachdem diese Zeitspanne verstrichen ist, wird die Spannungsversorgungseinheit ausreichend aufgeladen sein.
- Optional ermittelt die Steuereinheit 16.1, 16.2, 16.3, 54, 65, wie lange das Gasmessgerät 1.1, 1.2, 1.3 beim aktuellen Ladezustand verwendet werden kann, vorausgesetzt das Gasmessgerät 1.1, 1.2, 1.3 wird jetzt aus der Aufnahmeeinheit 10.1, 10.2, 10.3 entnommen, und seine Spannungsversorgungseinheit wird nicht weiter aufgeladen. Hierfür verwendet die Steuereinheit 16.1, 16.2, 16.3, 54, 65 den ermittelten aktuellen Ladezustand sowie einen vorgegebenen und bevorzugt vorab empirisch ermittelten Verbrauch pro Zeiteinheit an elektrischer Energie.
- Die Steuereinheit 16.1, 16.2, 16.3, 54, 65 prüft, ob die gemessene Zielgas-Konzentration um mehr als eine vorgegebene Toleranz von der vorgegebenen Zielgas-Konzentration des Prüfgases im Behälter 40 abweicht. Falls die Abweichung größer als die Toleranz ist, so misst das Gasmessgerät 1.1, 1.2, 1.3 nicht korrekt, ist also nicht intakt.

In einer Ausgestaltung wird der Datensatz für ein Gasmessgerät 1.1, 1.2, 1.3 um den ermittelten aktuellen Zustand ergänzt, oder der Datensatz wird aktualisiert.

Die Steuereinheit 16.1, 16.2, 16.3, 54, 65 vermag die Anzeige- und Bedieneinheit 20 anzusteuern. Auf dem Bildschirm 21 werden abhängig von der Ansteuerung Informationen über eingesetzte Gasmessgeräte 1.1, 1.2, 1.3 ausgegeben.

Insbesondere wird eine entsprechende Information ausgegeben, falls ein eingesetztes Gasmessgerät 1.1, 1.2, 1.3 aktuell ein Zielgas nicht korrekt zu messen vermag. Insbesondere werden die tatsächliche Zielgas-Konzentration eines Prüfgases im Behälter 40 sowie diejenige Zielgas-Konzentration, die das Gasmessgerät 1.1, 1.2, 1.3 für eine Gasprobe mit diesem Prüfgas gemessen hat, ausgegeben. Optional wird dann, wenn die Spannungsversorgungseinheit eines Gasmessgeräts 1.1, 1.2, 1.3 einen zu geringen Ladezustand aufweist, angezeigt, wann diese Spannungsversorgungseinheit ausreichend aufgeladen sein wird. Optional wird angezeigt, wie lange die Spannungsversorgungseinheit eines Gasmessgeräts 1.1, 1.2, 1.3 aktuell dieses Gasmessgerät ausreichend mit elektrischer Energie zu versorgen vermag.

Die Steuereinheit 16.1, 16.2, 16.3, 54, 65 vermag jede Arretiereinheit 14.1, 14.2, 14.3 des Ausgabeautomaten 100 anzusteuern. Als Ergebnis der Ansteuerung ist die Arretiereinheit 14.1, 14.2, 14.3 im arretierenden Zustand oder im freigebenden Zustand. Außerdem vermag die Steuereinheit 16.1, 16.2, 16.3, 54, 65 das jeweilige Ventil in einem Kontaktelement 11.1, 11.2, 11.3 anzusteuern und dadurch eine Fluidverbindung zwischen einem Behälter 40 mit einem Prüfgas und einem eingesetzten Gasmessgerät 1.1, 1.2, 1.3 wahlweise zu versperren oder freizugeben.

In einer Ausgestaltung löst der Vorgang, dass ein Gasmessgerät 1.1, 1.2, 1.3 in einer Aufnahmeeinheit 10.1, 10.2, 10.3 eingesetzt ist, folgende Schritte aus: Die Steuereinheit 16.1, 16.2, 16.3, 54, 65 steuert das Ventil des zugeordneten Kontaktelements 11.1, 11.2, 11.3 an, das Ventil öffnet sich, und ein Prüfgas wird aus dem Behälter 40 in die Messkammer des gerade eingesetzten Gasmessgeräts 1.1, 1.2, 1.3 geleitet. Die Steuereinheit 16.1, 16.2, 16.3, 54, 65 vergleicht die Zielgas-Konzentration, die das eingesetzte Gasmessgerät 1.1, 1.2, 1.3 gemessen hat, mit der tatsächlichen Zielgas-Konzentration.

An einer Seitenwand des Gehäuses 101 ist ein Träger 67 dergestalt gelagert, dass der Träger 67 relativ zum Gehäuse 101 um eine Drehachse DA.3 zwischen zwei Endpositionen drehbar ist, vgl. Figur 8 bis Figur 10. In jeder Endposition rastet der Träger 67 ein. In den Träger 67 sind ein RFID-Lesegerät 60 sowie ein Scanner 66, beispielsweise ein Barcode-Scanner oder QR-Code-Scanner, eingelassen. Weil der Träger 67 drehbar ist, wird es einem Benutzer erleichtert, den Träger 67 in eine gewünschte Position zu drehen. Am Träger 67 ist weiterhin eine Hülse 69 angeordnet, vgl. Figur 10. Durch diese Hülse 69 hindurch ist ein Scanner-Kabel (nicht gezeigt) geführt, das beim Betrieb in die Steckdose 35 im Gehäuse 101 eingesteckt ist.

Das RFID-Lesegerät 60 und der Scanner 66 haben in eine Ausgestaltung die folgenden beiden Aufgaben:
- Zum einen können ein Benutzer, der ein Gasmessgerät 1.x entnehmen möchte, oder auch ein Wartungstechniker sich wahlweise mit dem RFID-Lesegerät 60 oder mit dem Scanner 66 autorisieren.
- Zum anderen kann ein Benutzer, der ein Gasmessgerät 1.y zurückgeben möchte, oder auch ein Wartungstechniker bewirken, dass das RFID-Lesegerät 60 oder der Scanner 66 eine maschinenlesbare Kennung auf einer Oberfläche dieses Gasmessgeräts 1.y liest.

Zu Beginn eines Einsatzes des Ausgabeautomaten 100 ist bevorzugt folgende Situation hergestellt: Jeder Verschluss 13.1, 13.2, 13.3 und jedes Kontaktelement 11.1, 11.2, 11.3 sind nach unten gedreht. Jeder Verschluss 13.1, 13.2, 13.3 ist arretiert. Mindestens einmal wird eine Abfolge mit den folgenden Schritten durchgeführt:
Eine Person möchte ein Gasmessgerät verwenden. Diese Person wird nachfolgend als "der Benutzer" bezeichnet. Der Benutzer autorisiert sich bei der Anzeige- und Bedieneinheit 20 und meldet sich dadurch an. Beispielsweise gibt der Benutzer eine Benutzerkennung und ein Passwort ein. Oder eine Kennung auf einem Mitarbeiterausweis oder ein biometrisches Merkmal wird ermittelt. Die nachfolgenden Schritte werden nur dann durchgeführt, wenn der Benutzer sich erfolgreich autorisiert hat.

Optional gibt der Benutzer mittels der Anzeige- und Bedieneinheit 20 vor, für welches Zielgas er ein Gasmessgerät benötigt. Optional gibt der Benutzer vor, für welche Zeitdauer er das Gasmessgerät längstens benutzen will. Optional gibt der Benutzer eine Kennzeichnung für eine Umgebungsbedingung bei einem beabsichtigten Einsatz des Gasmessgeräts vor, beispielsweise die größte zu erwartende Umgebungstemperatur oder Umgebungsfeuchte.

In einer Ausgestaltung ist in einem Datenspeicher für eine Menge von möglichen Benutzern jeweils ein Qualifikationsprofil hinterlegt. Dieses Qualifikationsprofil spezifiziert, welche Gasmessgeräte dieser Benutzer verwenden darf.

Das Ereignis, dass ein Benutzer sich erfolgreich autorisiert hat und die Ausgabe eines Gasmessgeräts wünscht, löst folgenden Schritt aus: Die Steuereinheit 16.1, 16.2, 16.3, 54, 65 sucht nach einem Gasmessgerät, das aktuell kumulativ folgende Eigenschaften aufweist:
- Das Gasmessgerät ist in eine Aufnahmeeinheit 10.1, 10.2, 10.3 eingesetzt.
- Das Gasmessgerät ist dazu ausgestaltet, die Konzentration desjenigen Zielgases zu messen, für das der Benutzer ein Gasmessgerät benötigt.
- Das Gasmessgerät ist intakt, vermag also die Konzentration des Zielgases ausreichend genau zu messen. Dies bedeutet: Das Gasmessgerät hat die Konzentration dieses Zielgases in einer Gasprobe von dem Prüfgas aus dem Behälter 40 gemessen, und die gemessene Zielgas-Konzentration weicht von der tatsächlichen Zielgas-Konzentration um nicht mehr als die vorgegebene absolute oder prozentuale Toleranz ab.
- Die Spannungsversorgungseinheit des Gasmessgeräts weist einen ausreichend hohen Ladezustand auf, also einen Ladezustand oberhalb der vorgegebenen unteren Schranke.
- In einer Ausgestaltung hat der Benutzer eine Höchstdauer für die Benutzung des Gasmessgeräts vorgegeben, und der Ladezustand des Gasmessgeräts reicht mindestens für diese Höchstdauer aus.
- In einer Ausgestaltung hat der Benutzer mindestens eine erwartete Umgebungsbedingung vorgegeben, und das Gasmessgerät ist für diese Umgebungsbedingung geeignet, kann also insbesondere bei der vorgegebenen Umgebungstemperatur oder Umgebungsfeuchte korrekt messen.
- In einer Ausgestaltung ist für den Benutzer, der sich an der Anzeige- und Bedieneinheit 20 autorisiert hat, ein Qualifikationsprofil hinterlegt. Der Benutzer ist gemäß dieses Qualifikationsprofil dafür befähigt und geeignet, das Gasmessgerät zu verwenden.

Falls ein Gasmessgerät diese Bedingungen kumulativ erfüllt, so ist es verwendbar.

Falls die Steuereinheit 16.1, 16.2, 16.3, 54, 65 kein verwendbares Gasmessgerät findet, so bewirkt die Steuereinheit 16.1, 16.2, 16.3, 54, 65, dass auf dem Bildschirm 21 eine entsprechende Fehlernachricht ausgegeben wird. Falls die Steuereinheit 16.1, 16.2, 16.3, 54, 65 mehrere verwendbare Gasmessgeräte findet, so wählt die Steuereinheit 16.1, 16.2, 16.3, 54, 65 automatisch eines aus, beispielsweise das mit dem aktuell höchsten Ladezustand oder mit der besten Konzentrations-Messung (das mit dem geringsten Fehler).

Nachfolgend wird beschrieben, welche Schritte durchgeführt werden, nachdem die Steuereinheit 16.1, 16.2, 16.3, 54, 65 ein verwendbares Gasmessgerät 1.x (x=1,2, ...) gefunden hat und bei mehreren verwendbaren Gasmessgeräten eines davon ausgewählt hat. Das Gasmessgerät 1.x ist in die Aufnahmeeinheit 10.x eingesetzt. Anfänglich verschließt der Verschluss 13.x die Aufnahmeeinheit 10.x, und die Arretiereinheit 14.x arretiert den Verschluss 13.x.

Die Steuereinheit 16.1, 16.2, 16.3, 54, 65 bewirkt, dass folgende Schritte durchgeführt werden:
- Die Arretiereinheit 14.x wird in den freigebenden Zustand überführt.
- Bevorzugt öffnet der Verschluss 13.x sich automatisch, springt beispielsweise auf. Alternativ wird auf dem Bildschirm 21 eine Meldung angezeigt, dass der Verschluss 13.x nunmehr nicht mehr arretiert, sondern freigegeben ist. Diese Meldung umfasst eine visuell wahrnehmbare Kennzeichnung des Verschlusses 13.x oder der Aufnahmeeinheit 10.x.
- Der Benutzer kann nunmehr das Kontaktelement 11.x nach oben drehen und das Gasmessgerät 1.x entnehmen.
- Geprüft wird, ob das Gasmessgerät 1.x aus der Aufnahmeeinheit 10.x entnommen worden ist. Sobald dies der Fall ist, wird der Datensatz für das Gasmessgerät 1.x entsprechend aktualisiert.

Bei der gerade beschriebenen Ausgestaltung wird sichergestellt, dass der Benutzer nur ein verwendbares Gasmessgerät entnehmen kann. Eine bevorzugte Ausgestaltung ermöglicht es einem Wartungstechniker, ein Gasmessgerät zu entnehmen, welches nicht notwendigerweise intakt ist. Ein Wartungstechniker ist eine Person, die ein Gasmessgerät entnehmen, überprüfen und bei Bedarf reparieren oder ersetzen darf und kann.

Der Wartungstechniker autorisiert sich am Ausgabeautomaten 100. In einer ersten Ausgestaltung löst der Schritt, dass sich ein Wartungstechniker autorisiert hat, folgenden Schritt aus: Die Steuereinheit 16.1, 16.2, 16.3, 54, 65 bewirkt durch eine entsprechende Ansteuerung, dass jede Arretiereinheit 14.1, 14.2, 14.3 in den freigebenden Zustand überführt wird. Dadurch lässt sich jeder Verschluss 13.1, 13.2, 13.3 öffnen. Der Wartungstechniker kann jedes eingesetzte Gasmessgerät 1.1, 1.2, 1.3 entnehmen.

In einer zweiten Ausgestaltung ermittelt die Steuereinheit 16.1, 16.2, 16.3, 54, 65 dann, wenn ein Wartungstechniker sich autorisiert hat, jedes eingesetzte Gasmessgerät, das mindestens eine der folgenden Eigenschaften aufweist:
- Das Gasmessgerät vermag eine Zielgas-Konzentration nicht korrekt zu messen. Insbesondere hat das Gasmessgerät die Konzentration eines Zielgases in einer Gasprobe gemessen, wobei diese Gasprobe vom Prüfgas aus dem Behälter 40 stammt, und die gemessene Zielgas-Konzentration weicht um mehr als die vorgegebene Toleranz von der tatsächlichen Zielgas-Konzentration im Prüfgas ab.
- Die Steuereinheit 16.1, 16.2, 16.3, 54, 65 hat festgestellt, dass das Gasmessgerät einen sonstigen Fehler aufweist, beispielsweise seine Spannungsversorgungseinheit oder seine Ausgabeeinheit oder seine Alarmeinheit defekt sind.

Gemäß der zweiten Ausgestaltung bewirkt die Steuereinheit 16.1, 16.2, 16.3, 54, 65 Folgendes: Der jeweilige Verschluss jeder Aufnahmeeinheit, in die ein defektes Gasmessgerät eingesetzt ist, wird dearretiert und dadurch freigegeben. Der Wartungstechniker kann das oder jedes defekte Gasmessgerät entnehmen.

In einer dritten Ausgestaltung wählt der Wartungstechniker eine Aufnahmeeinheit 10.z (z=1,2, ...) aus, beispielsweise durch eine entsprechende Benutzereingabe auf dem Bildschirm 21. Die Arretiereinheit 14.z wird in den freigebenden Zustand überführt. Der Verschluss 13.z lässt sich öffnen oder springt auf. Der Wartungstechniker kann aus der Aufnahmeeinheit 10.z das eingesetzte Gasmessgerät 1.z entnehmen.

Nach einer Benutzung gibt ein Benutzer ein Gasmessgerät 1.y zurück, indem er es in eine Aufnahmeeinheit 10.y (y=1, 2, ...) des Ausgabeautomaten 100 einsetzt. Um das Gasmessgerät 1.y einzusetzen, dreht der Benutzer den zugeordneten Verschluss 13.y nach oben, oder der Verschluss 13.y springt von allein auf. Außerdem dreht der Benutzer das Kontaktelement 11.y nach oben.

Nach dem Einsetzen verschließt der Benutzer den zugeordneten Verschluss 13.y. Der Schritt, den Verschluss 13.y zu verschließen, bewirkt aufgrund der oben beschriebenen mechanischen Kopplung, dass auch das Kontaktelement 11.y verschlossen wird. Sobald der Verschluss 13.y verschlossen ist, bewirken die Steuereinheit 16.1, 16.2, 16.3, 54, 65 oder auch ein Rückstellelement, dass die zugeordnete Arretiereinheit 14.y den Verschluss 13.y arretiert.

In einer Ausgestaltung ist dann, wenn in eine Aufnahmeeinheit 10.y kein Gasmessgerät eingesetzt ist, der zugeordnete Verschluss 13.y dearretiert. In einer abweichenden Ausgestaltung arretiert hingegen die Arretiereinheit 14.y zunächst den Verschluss 13.y für die freie Aufnahmeeinheit 10.y.

Gemäß einer Realisierungsform der alternativen Ausgestaltung bringt der Benutzer zunächst das Gasmessgerät 1.y, das er zurückgeben möchte, in die Nähe des RFID-Lesegeräts 60 oder des Scanners 66. Das RFID-Lesegerät 60 oder der Scanner 66 lesen eine maschinenlesbare Kennung auf einer Oberfläche des Gasmessgeräts 1.y. Falls eine solche Kennung nicht gefunden wird oder nicht gelesen werden kann, so bewirkt die Steuereinheit 65, dass eine entsprechende Fehlermeldung ausgegeben wird.

In einer anderen Realisierungsform umfassen der Ausgabeautomat 100 und jedes Gasmessgerät jeweils eine Kommunikationseinheit für eine drahtlose Datenkommunikation über eine relativ geringe Entfernung, beispielsweise per Bluetooth Low Energy (BLE) oder allgemein Near Field Communication (NFC). Die Kommunikationseinheit des Ausgabeautomaten 100 empfängt eine Nachricht von einem Gasmessgerät, welches in die Nähe des Ausgabeautomaten 100 verbracht wurde. Diese Nachricht umfasst eine eindeutige Kennung des Gasmessgeräts.

Sobald ein zurückzugebendes Gasmessgerät 1.y erkannt worden ist, sucht die Steuereinheit 65 automatisch nach einer passenden Aufnahmeeinheit 10.y.

Insbesondere sucht sie nach einer freien Aufnahmeeinheit 10.y mit passenden Abmessungen und einer passenden elektrischen Koppelstelle. Falls keine passende Aufnahmeeinheit gefunden wird, bewirkt die Steuereinheit 65 wiederum, dass eine entsprechende Fehlermeldung ausgegeben wird. Falls eine passende Aufnahmeeinheit 10.y gefunden wird, so bewirkt die Steuereinheit 65, dass die zugeordnete Arretiereinheit 14.y in den freigegebenen Zustand überführt wird. Der zugeordnete Verschluss 13.y lässt sich öffnen oder springt von allein auf.

Der Ausgabeautomaten 100 ist wenigstens zeitweise mit dem Behälter 40 verbunden, vgl. Figur 1. In einer Ausgestaltung löst der Schritt, dass das Gasmessgerät 1.y in die Aufnahmeeinheit 10.y eingesetzt wird, das Kontaktelement 11.y und der Verschluss 13.y zur Aufnahmeeinheit 10.y hin gedreht werden und die Arretiereinheit 14.y in den arretierenden Zustand überführt wird, folgenden Schritt aus: Ein Prüfgas aus dem Behälter 40 wird zum Kontaktelement 11.y und weiter zur Messkammer des eingesetzten Gasmessgeräts 1.y geleitet. Das Gasmessgerät 1.y misst die Zielgas-Konzentration im Prüfgas, und die gemessene wird mit der tatsächlichen Zielgas-Konzentration verglichen. Dadurch wird das Gasmessgerät 1.y unmittelbar nach dem Einsetzen überprüft. Bevorzugt wird für diese Überprüfung das oben genannte Ventil im Kontaktelement 11.y geöffnet, und dadurch wird das Gaszuführelement 12.y freigegeben.

Weiter oben wurde mit Bezug auf Figur 2 eine Datenbank 55 erwähnt, in der für jedes Gasmessgerät jeweils ein Datensatz angelegt ist. Der gerade beschriebene Vorgang, dass das RFID-Lesegerät 60 oder der Scanner 66 die Kennung eines Gasmessgeräts, welches ein Benutzer zurückgeben will, gelesen hat, lösen in einer Ausgestaltung folgenden Schritte aus: Der Datensatz für dieses Gasmessgerät 1.y in der Datenbank 55 wird entsprechend aktualisiert. Mindestens wird der Datensatz um die Information ergänzt, dass das Gasmessgerät 1.y sich nunmehr im Ausgabeautomaten 100 befindet, bevorzugt zusätzlich um die Information, an welchem Zeitpunkt das Gasmessgerät 1.y in die Aufnahmeeinheit 10.y eingesetzt worden ist, und / oder um die Information, in welche Aufnahmeeinheit ist eingesetzt worden ist

Häufig entnimmt ein Benutzer dem Ausgabeautomaten 100 ein Gasmessgerät, um es in einer für Menschen möglicherweise gefährlichen Umgebung einzusetzen. In vielen Fällen nimmt das Gasmessgerät, insbesondere ein Gehäuse des Gasmessgeräts, hierbei mindestens ein Fluid auf, beispielsweise einen langkettigen Kohlenwasserstoff. Als Folge dieses oft zwangsläufigen Ereignisses tritt dann, wenn das Gasmessgerät 1.y wieder in eine Aufnahmeeinheit des Ausgabeautomaten 100 eingesetzt ist, Gas aus dem Gasmessgerät 1.y aus. In einer Realisierungsform vermag ein Sensor, der dauerhaft der Aufnahmeeinheit zugeordnet ist, dieses Gas zu detektieren. Die Steuereinheit 16.1, 16.2, 16.3, 54, 65 bewirkt, dass eine entsprechende Meldung erzeugt und ausgegeben wird.

In einer Ausgestaltung löst der Schritt, ein Gasmessgerät 1.y in eine Aufnahmeeinheit 10.y einzusetzen, folgenden Schritte aus: Das Gasmessgerät wird automatisch desinfiziert und / oder gereinigt, beispielsweise mittels einem Spülgases oder mittels desinfizierender elektromagnetischer Strahlung, insbesondere Ultraviolettstrahlung. Diese Ausgestaltung reduziert die Gefahr, dass ein eingesetztes kontaminiertes Gasmessgerät 1.y andere Gasmessgeräte im Ausgabeautomaten 100 kontaminiert. Während das Gasmessgerät 1.y desinfiziert und / oder gereinigt wird, arretiert bevorzugt die Arretiereinheit 14.y den Verschluss 13.y.

Die nachfolgende Ausgestaltung berücksichtigt die Möglichkeit, dass ein zurückzugebendes Gasmessgerät 1.y mechanisch beschädigt ist. Die häufigsten Arten mechanischer Beschädigung sind die folgende:
- Der Bildschirm ist gebrochen oder zerkratzt.
- Eine Halterung, mit der sich das Gasmessgerät an der Schutzbekleidung eines Benutzers befestigen lässt, ist beschädigt.
- Im Gehäuse ist ein Riss aufgetreten.
- Das Gasmessgerät ist verschmutzt, insbesondere verschmiert.

In einer Ausgestaltung wird der Benutzer, der das Gasmessgerät zurückgeben will, um eine Eingabe gebeten, ob das Gasmessgerät beschädigt ist oder nicht. Der Ausgabeautomat 100 erfasst eine entsprechende Benutzereingabe. Die nachfolgende alternative Ausgestaltung erspart die Notwendigkeit, dass der Benutzer eine solche Prüfung und Eingabe vornimmt.

Gemäß dieser alternativen Ausgestaltung umfasst der Ausgabeautomat 100 eine Kamera und eine signalverarbeitende Bildauswerteeinheit. Beispielsweise ist der Scanner 66 als eine Kamera ausgestaltet oder umfasst eine Kamera. Die Bildauswerteeinheit kann ein Bestandteil der Steuereinheit 65 sein. In einer Ausgestaltung vermag diese Kamera einen Barcode oder QR-Code oder auch eine alphanumerische Kennung zu lesen und zu dekodieren. Der Barcode oder QR-Code oder die alphanumerische Kennung kennzeichnet das Gasmessgerät 1.y eindeutig. Möglich ist auch, dass ein NFC-Lesegerät, insbesondere das RFID-Lesegerät 60, einen entsprechenden Chip im Gasmessgerät 1.y ausliest und dadurch die Kennung des Gasmessgeräts 1.y ermittelt. In beiden Ausgestaltungen wird die Kamera verwendet, um automatisch eine mechanische Beschädigung des Gasmessgeräts 1.y zu detektieren.

Die Kamera 66 erzeugt Bilder von dem zurückzugebenden Gasmessgerät 1.y, welches sich außerhalb des Ausgabeautomaten 100 und ausreichend nahe zur Kamera befindet. Beispielsweise umfasst der Ausgabeautomat 100 eine Platte oder Schale oder sonstige Aufnahme. Das RFID-Lesegerät 60 kann selber in eine solche Platte integriert sein. Falls das zurückzugebende Gasmessgerät 1.y auf diese Auflage gelegt wird, so ist es richtig relativ zur Kamera 66 positioniert. Die Kamera 66 erzeugt mindestens ein Bild vom Gasmessgerät 1.y. Bevorzugt erzeugt die Kamera 66 ein Bild von der Vorderseite und ein Bild von der Rückseite des Gasmessgeräts 1.y, wobei der Benutzer das Gasmessgerät 1.y so positioniert, dass die Vorderseite bzw. die Rückseite zur Kamera 66 zeigt.

Die Bildauswerteeinheit wertet diese Bilder aus und entscheidet automatisch, ob in mindestens einem Bild eine mechanische Beschädigung des Gasmessgeräts 1.y zu erkennen ist. Falls ja, so wird eine entsprechende Meldung ausgegeben, und / oder der Datensatz in der Datenbank 55 für dieses Gasmessgerät 1.y wird entsprechend aktualisiert. Bevorzugt umfasst die Bildauswerteeinheit einen trainierten Klassifikator. Dieser Klassifikator wurde vorab mithilfe eines Verfahrens des maschinellen Lernens trainiert. Besonders bevorzugt wird der Klassifikator mithilfe einer annotierten Stichprobe trainiert. Diese Stichprobe umfasst eine Vielzahl von Bildern, wobei jedes Bild jeweils ein Gasmessgerät zeigt und dem Bild eine Annotation zugeordnet ist. Die andere Annotation beschreibt, ob das im Bild gezeigte Gasmessgerät eine mechanische Beschädigung aufweist oder nicht, und wenn ja, welche mechanische Beschädigung vorliegt.

In einer Ausgestaltung ist mindestens eine Aufnahmeeinheit eine Waage zugeordnet. Der Schritt, dass ein Gasmessgerät 1.y in dieser Aufnahmeeinheit eingesetzt worden ist, löst folgenden Schritt aus: Die Waage misst das Gewicht des eingesetzten Gasmessgeräts 1.y. Das gemessene Gewicht wird mit einem vorgegebenen Soll-Gewicht verglichen, wobei dieses Soll-Gewicht beispielsweise ein Bestandteil des Datensatzes für dieses Gasmessgerät 1.y in der Datenbank 55 ist. Falls das gemessene Gewicht erheblich vom Soll-Gewicht abweicht, so ist dies ein Indiz für einen Fehler. Beispielsweise fehlt dem zurückgegebenen Gasmessgerät 1.y ausreichend schweres Teil, insbesondere eine Batterie.

### Bezugszeichenliste

| | |
|---|---|
| 1.1, 1.2, 1.3 | Gasmessgerät, befindet sich in der Aufnahmeeinheit 10.1, 10.2, 10.3, umfasst den Gaseinlass 2.1, 2.2, 2.3, die Anzeigeeinheit 3.1, 3.2, 3.3 und die Bedienelemente 4.1, 4.2, 4.3 sowie eine Messkammer, einen Sensor und eigene Spannungsversorgungseinheit |
| 2.1, 2.2, 2.3 | Gaseinlass des Gasmessgeräts 1.1, 1.2, 1.3 |
| 3.1, 3.2, 3.3 | Anzeigeeinheit des Gasmessgeräts 1.1, 1.2, 1.3 |
| 4.1, 4.2, 4.3 | Bedienelemente des Gasmessgeräts 1.1, 1.2, 1.3 |
| 10.1, 10.2, 10.3 | Aufnahmeeinheit, vermag ein Gasmessgerät 1.1, 1.2, 1.3 aufzunehmen, lässt sich von einem Verschluss 13.1, 13.2, 13.3 verschließen, umfasst das Kontaktelement 11.1, 11.2, 11.3 und die elektrische Kontaktierung 51.1, 51.2, 51.3 |
| 11.1, 11.2, 11.3 | Kontaktelement der Aufnahmeeinheit 10.1, 10.2, 10.3, nimmt das Gaszuführelement 12.1, 12.2, 12.3 auf, relativ zum Gehäuse 101 um die Drehachse DA.2 drehbar |
| 12.1, 12.2, 12.3 | Gaszuführelement im Kontaktelement 11.1, 11.2, 11.3, mit der Gasleitung 30 verbunden, gehört zur Fluidführungs-Anordnung |
| 13.1, 13.2, 13.3 | Verschluss für die Aufnahmeeinheit 10.1, 10.2, 10.3, relativ zum Gehäuse 101 um die Drehachse DA.1 drehbar, umfasst den Vorsprung 17.1, 17.2, 17.3, das Fenster 19.1, 19.2, 19.3 und das Abstandselement 59.1, 59.2, 59.3 |
| 14.1, 14.2, 14.3 | Arretiereinheit für den Verschluss 13.1, 13.2, 13.3, umfasst die Aussparung 18.1, 18.2, 18.3 sowie einen Arretierkörper, ein Stellglied und optional ein Rückstellelement, lässt sich in einen arretierenden und in einen freigebenden Zustand überführen, umfasst gemäß der zweiten Realisierungsform das mechanisches Schloss 64.1, 64.2, 64.3 |
| 15.1, 15.2, 15.3 | Spannungssensor der Aufnahmeeinheit 10.1, 10.2, 10.3, misst den Ladezustand der Spannungsversorgungseinheit eines eingesetzten Gasmessgeräts 1.1, 1.2, 1.3 |
| 16.1, 16.2, 16.3 | Auswerteeinheit der Aufnahmeeinheit 10.1, 10.2, 10.3, vergleicht eine gemessene mit einer tatsächlichen Zielgas-Konzentration |
| 17.1, 17.2, 17.3 | Vorsprung am Verschluss 13.1, 13.2, 13.3, greift in die Aussparung 18.1, 18.2, 18.3 ein, wenn der Verschluss 13.1, 13.2, 13.3 in der verschließenden Position ist |
| 18.1, 18.2, 18.3 | Aussparung in der Arretiereinheit 14.1, 14.2, 14.3, nimmt den Vorsprung 17.1, 17.2, 17.3 auf, wenn der Verschluss 13.1, 13.2, 13.3 in der verschließenden Position ist |
| 19.1, 19.2, 19.3 | Fenster im Verschluss 13.1, 13.2, 13.3, durch das hindurch ein eingesetztes Gasmessgerät 1.1, 1.2, 1.3 sichtbar ist |
| 20 | Anzeige- und Bedieneinheit, umfasst den Bildschirm 21, in einer Ausgestaltung den Bildschirm 70 sowie den Einschaltknopf 22, fungiert als die Eingabeeinheit |
| 21 | berührungssensitiver Bildschirm (Touch Screen) der Anzeige- und Bedieneinheit 20 |
| 22 | Einschaltknopf der Anzeige- und Bedieneinheit 20 |
| 25.1, 25.2 | Verschluss-Bauteil gemäß der ersten Realisierungsform der Erfindung, umgibt die Aufnahmeeinheit 10.1, 10.2, umfasst den Verschluss 13.1, 13.2, die Arretiereinheit 14.1, 14.2 und die Halterung 56.1, 56.2 |
| 26.1, 26.2, 26.3 | Aufnahme-Modul gemäß der zweiten Realisierungsform, nimmt die Aufnahmeeinheit 10.1, 10.2, 10.3 auf, umfasst den Verschluss 13.1, 13.2, 13.3, die Arretiereinheit 14.1, 14.2, 14.3 und die Halterung 56.1, 56.2, 56.3 |
| 30 | Gasleitung, verbindet den Gaseinlass 31 mit den Gaszuführelementen 12.1, 12.2, 12.3, gehört zur Fluidführungs-Anordnung |
| 31 | Gaseinlass im Gehäuse 101, Anfang der Gasleitung 30 |
| 32 | weiterer Gaseinlass im Gehäuse 101 |
| 33 | Anschluss im Gehäuse 101 für ein Ethernet-Kabel zur Datenbank 55 |
| 34 | Steckdose im Gehäuse 101 für eine Leitung, die den Ausgabeautomaten 100 mit einem stationären Spannungsversorgungsnetz verbindet |
| 35 | Steckdose im Gehäuse 101 für ein Scanner-Kabel |
| 40 | Behälter in Form einer Flasche mit Prüfgas, mit der Leitung 41 verbunden |
| 41 | Fluidführungseinheit in Form einer Leitung, die den Behälter 40 mit dem Gaseinlass 31 verbindet |
| 50.1, 50.2, 50.3 | federbelasteter Stab, öffnet den Verschluss 13.1, 13.2, 13.3, überträgt ein Schließen des Verschlusses 13.1, 13.2, 13.3 auf das Kontaktelement 11.1, 11.2, 11.3, trägt an seinem freien Ende die Walze 58.1, 58.2, 58.3, fungiert als Öffnungselement |
| 51.1, 51.2, 51.3 | elektrische Kontaktierung in der Aufnahmeeinheit 10.1, 10.2, 10.3, kontaktiert elektrisch ein eingesetztes Gasmessgerät 1.1, 1.2, 1.3 |
| 53.1, 53.2, 53.3 | Walze, drehbar am freien Ende des Abstandselements 59.1, 59.2, 59.3 gelagert |
| 54 | Steuergerät des Ausgabeautomaten 100 |
| 55 | Datenbank mit Datensätzen für die Gasmessgeräte, über ein Ethernet-Kabel mit der Prüfstation 80 verbunden |
| 56.1, 56.2, 56.3 | Halterung, an der der Verschluss 13.1, 13.2, 13.3 drehbar befestigt ist, ist mechanisch mit dem Gehäuse 101 verbunden, hält den Stab 50.1, 50.2, 50.3, gehört zum Verschluss-Bauteil 25.1, 25.2 und zum Aufnahme-Modul 26.1, 26.2, 26.3 |
| 57.1, 57.2, 57.3 | Aussparung in der Halterung 56.1, 56.2, 56.3 für den Stab 50.1, 50.2, 50.3 |
| 58.1, 58.2, 58.3 | Walze, drehbar am freien Ende des Stabs 50.1, 50.2, 50.3 gelagert |
| 59.1, 59.2, 59.3 | Abstandselement, am Verschluss 13.1, 13.2, 13.3 befestigt, umfasst die Walze 58.1, 58.2, 58.3, dreht das Kontaktelement 11.1, 11.2, 11.3 nach unten |
| 60 | RFID-Lesegerät, am Träger 67 befestigt |
| 61 | Schutzklappe für die Anzeige- und Bedieneinheit 20, nimmt den Bildschirm 70 auf, umfasst das Fenster 62, ist relativ zum Gehäuse 101 um die Drehachse DA.1 drehbar |
| 62 | Fenster der Schutzklappe 61, durch das hindurch der Bildschirm 21 sichtbar ist |
| 63 | mechanisches Schloss für die Schutzklappe 61 |
| 64.1, 64.2, 64.3 | mechanisches Schloss für den Verschluss 13.1, 13.2, 13.3 |
| 65 | Steuereinheit, führt die Funktionen des Steuergeräts 54 und der Auswerteeinheiten 16.1, 16.2, 16.3 aus |
| 66 | Scanner, beispielsweise Barcode-Scanner, am Träger 67 befestigt |
| 67 | Träger für das RFID-Lesegerät 60 und den Scanner 66, seitlich am Gehäuse 101 angeordnet, relativ zum Gehäuse 101 und die Drehachse DA.3 drehbar gelagert |
| 69 | Hülse am Träger 67, durch das hindurch ein Scanner-Kabel geführt ist |
| 70 | berührungssensitiver Bildschirm, in die Schutzklappe 61 eingelassen |
| 71 | Scharnier, mit dem die Schutzklappe 61 am Gehäuse 101 befestigt ist |
| 80 | Prüfstation, umfasst die Anzeige- und Bedieneinheit 20, die Aufnahmeeinheiten 10.1, 10.2, 10.3, die Gasleitung 30, die Gaseinlässe 31, 32 und das Gehäuse 101 |
| 100 | Ausgabeautomat, umfasst die Prüfstation 80, die Verschlüsse 13.1, 13.2, 13.3, die Arretiereinheiten 14.1, 14.2, 14.3, die Spannungssensoren 15.1, 15.2, 15.3 sowie gemäß der ersten Realisierungsform die Auswerteeinheiten 16.1, 16.2, 16.3 und das Steuergerät 54 und gemäß der zweiten Realisierungsform die Steuereinheit 65 |
| 101 | Gehäuse der Prüfstation 80, nimmt die Gaseinlässe 31 und 32, den Anschluss 33 und die Steckdosen 34 und 35 auf |
| DA.1 | Drehachse, um die der Verschluss 13.1, 13.2, 13.3 relativ zum Gehäuse 101 drehbar ist, parallel zu den Drehachsen DA.2 und DA.3 |
| DA.2 | Drehachse, um die das Kontaktelement 11.1, 11.2, 11.3 relativ zum Gehäuse 101 drehbar ist |
| DA.3 | Drehachse, um die der Träger 67 relativ zum Gehäuse 101 drehbar gelagert ist |

## Patentansprüche

1. Ausgabeautomat (100) für ein Gasmessgerät (1.1, 1.2, 1.3),
wobei der Ausgabeautomat (100)
- eine Aufnahmeeinheit (10.1, 10.2, 10.3),
- einen Verschluss (13.1, 13.2, 13.3), der relativ zur Aufnahmeeinheit (10.1, 10.2, 10.3) zwischen einer verschließenden Position und einer geöffneten Position hin- und herbeweglich ist,
- eine Arretiereinheit (14.1, 14.2, 14.3), die in einen arretierenden Zustand und in einen freigebenden Zustand überführbar ist,
- einen Gaseinlass (31, 32),
- eine Fluidführungs-Anordnung (30, 11.1, 11.2, 11.3),
- eine Eingabeeinheit (20) und
- eine signalverarbeitende Steuereinheit (16.1, 16.2, 16.3, 54, 65)
umfasst,
wobei sich in die Aufnahmeeinheit (10.1, 10.2, 10.3) ein Gasmessgerät (1.1, 1.2, 1.3) einsetzen und wieder entnehmen lässt,
wobei die Fluidführungs-Anordnung (30, 11.1, 11.2, 11.3) den Gaseinlass (31, 32) mit der Aufnahmeeinheit (10.1, 10.2, 10.3) dergestalt verbindet,
dass dann, wenn ein Gasmessgerät (1.1, 1.2, 1.3) in die Aufnahmeeinheit (10.1, 10.2, 10.3) eingesetzt ist, die Fluidführungs-Anordnung (30, 11.1, 11.2, 11.3) eine Fluidverbindung zwischen dem Gaseinlass (31, 32) und dem eingesetzten Gasmessgerät (1.1, 1.2, 1.3) herstellt,
wobei der Verschluss (13.1, 13.2, 13.3) in der verschließenden Position verhindert, dass sich ein Gasmessgerät (1.1, 1.2, 1.3) aus der Aufnahmeeinheit (10.1, 10.2, 10.3) entnehmen lässt, und in der geöffneten Position die Entnahme eines Gasmessgeräts (1.1, 1.2, 1.3) ermöglicht,
wobei die Arretiereinheit (14.1, 14.2, 14.3) im arretierenden Zustand dann, wenn der Verschluss (13.1, 13.2, 13.3) in der verschließenden Position ist, eine Überführung des Verschlusses (13.1, 13.2, 13.3) in die geöffnete Position verhindert und diese Überführung im freigebenden Zustand ermöglicht,
wobei die Eingabeeinheit (20) dazu ausgestaltet ist, eine Benutzereingabe zu erfassen, welche die Information umfasst, dass die Ausgabe eines Gasmessgeräts (1.1, 1.2, 1.3) gewünscht ist,
wobei die Steuereinheit (16.1, 16.2, 16.3, 54, 65) dazu ausgestaltet ist, als Reaktion auf die Erfassung einer Benutzereingabe zu prüfen, ob in die Aufnahmeeinheit (10.1, 10.2, 10.3) ein verwendbares Gasmessgerät (1.1, 1.2, 1.3) eingesetzt ist,
wobei ein Gasmessgerät (1.1, 1.2, 1.3) nur dann verwendbar ist, wenn es intakt ist, und wobei es intakt ist, wenn es die Konzentration eines vorgegebenen Zielgases bis auf eine vorgegebene Toleranz korrekt zu messen vermag,
wobei die Steuereinheit (16.1, 16.2, 16.3, 54, 65) weiterhin dazu ausgestaltet ist, dann, wenn in die Aufnahmeeinheit (10.1, 10.2, 10.3) ein verwendbares Gasmessgerät (1.1, 1.2, 1.3) eingesetzt ist,
zu bewirken, dass die Arretiereinheit (14.1, 14.2, 14.3) für den Verschluss (13.1, 13.2, 13.3) in den freigebenden Zustand überführt wird.

2. Ausgabeautomat (100) nach Anspruch 1,
**dadurch gekennzeichnet, dass**
die Aufnahmeeinheit (10.1, 10.2, 10.3) ein Öffnungselement (50.1, 50.2, 50.3) umfasst,
welches dazu ausgestaltet ist, den Verschluss (13.1, 13.2, 13.3) aus der verschließenden Position in die geöffnete Position zu bewegen.

3. Ausgabeautomat (100) nach einem der vorhergehenden Ansprüche,
**dadurch gekennzeichnet, dass**
der Ausgabeautomat (100) einen Spannungssensor (15.1, 15.2, 15.3) umfasst und die Aufnahmeeinheit (10.1, 10.2, 10.3) eine elektrische Kontaktierung umfasst,
wobei dann, wenn ein Gasmessgerät (1.1, 1.2, 1.3) in die Aufnahmeeinheit (10.1, 10.2, 10.3) eingesetzt ist,
mithilfe der elektrischen Kontaktierung eine elektrische Verbindung zwischen einer Spannungsversorgungseinheit des eingesetzten Gasmessgeräts (1.1, 1.2, 1.3) und dem Spannungssensor (15.1, 15.2, 15.3) hergestellt oder wenigstens zeitweise herstellbar ist,
wobei der Spannungssensor (15.1, 15.2, 15.3) dazu ausgestaltet ist, den aktuellen Ladezustand der Spannungsversorgungseinheit des eingesetzten Gasmessgeräts (1.1, 1.2, 1.3) zu messen, und
wobei die Steuereinheit (16.1, 16.2, 16.3, 54, 65) dazu ausgestaltet ist, dann zu entscheiden, dass das eingesetzte Gasmessgerät (1.1, 1.2, 1.3) aktuell nicht verwendbar ist, wenn der gemessene Ladezustand kleiner als eine vorgegebene Ladezustands-Schranke ist.

4. Ausgabeautomat (100) nach einem der vorhergehenden Ansprüche,
**dadurch gekennzeichnet, dass**
die erfasste Benutzereingabe zusätzlich eine Information über eine gewünschte Eigenschaft eines Gasmessgeräts (1.1, 1.2, 1.3) umfasst und
die Steuereinheit (16.1, 16.2, 16.3, 54, 65) dazu ausgestaltet ist,
- zu prüfen, ob in die Aufnahmeeinheit (10.1, 10.2, 10.3) ein Gasmessgerät (1.1, 1.2, 1.3) eingesetzt ist, welches die gemäß der Benutzereingabe gewünschte Eigenschaft aufweist, und
- das eingesetzte Gasmessgerät (1.1, 1.2, 1.3) nur dann als verwendbar einzustufen, wenn es intakt ist und die gewünschte Eigenschaft aufweist.

5. Ausgabeautomat (100) nach einem der vorhergehenden Ansprüche,
**dadurch gekennzeichnet, dass**
die Aufnahmeeinheit (10.1, 10.2, 10.3) ein Kontaktelement (11.1, 11.2, 11.3) umfasst, welches relativ zu einem in die Aufnahmeeinheit (10.1, 10.2, 10.3) eingesetzten Gasmessgerät (1.1, 1.2, 1.3) zwischen einer kontaktierenden und einer beabstandeten Position hin- und herbeweglich ist,
wobei dann, wenn das Kontaktelement (11.1, 11.2, 11.3) in der kontaktierenden Position ist, die Fluidführungs-Anordnung (30, 11.1, 11.2, 11.3) eine Fluidverbindung zwischen dem Gaseinlass (31, 32) und dem eingesetzten Gasmessgerät (1.1, 1.2, 1.3) herstellt,
wobei dann, wenn das Kontaktelement (11.1, 11.2, 11.3) sich in der beabstandeten Position befindet, diese Fluidverbindung unterbrochen ist, und
wobei das Kontaktelement (11.1, 11.2, 11.3) dergestalt mit dem Verschluss (13.1, 13.2, 13.3) mechanisch gekoppelt ist, dass eine Bewegung des Verschlusses (13.1, 13.2, 13.3) von der geöffneten Position in die verschließende Position bewirkt,
dass das Kontaktelement (11.1, 11.2, 11.3) von der beabstandeten in die kontaktierende Position überführt wird.

6. Ausgabeautomat (100) nach einem der vorhergehenden Ansprüche,
**dadurch gekennzeichnet, dass**
der Ausgabeautomat (100) ein manuell betätigbares Betätigungselement umfasst,
wobei das Betätigungselement mit der Arretiereinheit (14.1, 14.2, 14.3) dergestalt verbunden ist,
dass eine Betätigung des Betätigungselements bewirkt, dass die Arretiereinheit (14.1, 14.2, 14.3) in den freigebenden Zustand überführt wird.

7. Ausgabeautomat (100) nach einem der vorhergehenden Ansprüche,
**dadurch gekennzeichnet, dass**
der Ausgabeautomat (100) eine Kamera (66) und eine signalverarbeitende Bildauswerteeinheit (65) umfasst,
wobei die Kamera (66) dazu ausgestaltet ist, von einem Gasmessgerät (1.1, 1.2, 1.3) ein Bild zu erzeugen,
wobei bevorzugt die Kamera (66) dergestalt positioniert ist, dass das Bild das Gasmessgerät (1.1, 1.2, 1.3) zeigt, während das Gasmessgerät (1.1, 1.2, 1.3) sich außerhalb des Ausgabeautomaten (100) befindet, und
wobei die Bildauswerteeinheit (65) dazu ausgestaltet ist, durch Auswertung des Bildes des Gasmessgeräts (1.1, 1.2, 1.3) automatisch zu entscheiden, ob das Gasmessgerät (1.1, 1.2, 1.3) eine optisch erkennbare, insbesondere eine mechanische Beschädigung aufweist oder nicht.

8. Ausgabeautomat (100) nach einem der vorhergehenden Ansprüche,
**dadurch gekennzeichnet, dass**
der Ausgabeautomat (100) dazu ausgestaltet ist, zu bewirken oder wenigstens zu ermöglichen, dass eine Gasprobe vom Gaseinlass (31, 32) durch die Fluidverbindung hindurch zu einem Gasmessgerät (1.1, 1.2, 1.3), das in die Aufnahmeeinheit (10.1, 10.2, 10.3) eingesetzt ist, fließt,
wobei die Gasprobe das Zielgas mit einer vorgegebenen Zielgas-Konzentration aufweist, und
die Steuereinheit (16.1, 16.2, 16.3, 54, 65) dazu ausgestaltet ist,
- zu bewirken, dass das eingesetzte Gasmessgerät (1.1, 1.2, 1.3) die Zielgas-Konzentration in der Gasprobe misst,
- die gemessene Zielgas-Konzentration mit einer vorgegebenen Zielgas-Konzentration zu vergleichen und
- dann zu entscheiden, dass das Gasmessgerät (1.1, 1.2, 1.3) nicht intakt ist, wenn die gemessene Zielgas-Konzentration von der vorgegebenen Zielgas-Konzentration um mehr als eine vorgegebene Toleranz abweicht.

9. Ausgabeautomat (100) nach Anspruch 8,
**dadurch gekennzeichnet, dass**
die Steuereinheit (16.1, 16.2, 16.3, 54, 65) dazu ausgestaltet ist,
- die Messung der Zielgas-Konzentration,
- den Vergleich der gemessenen mit der vorgegebenen Zielgas-Konzentration und
- die Entscheidung
als Reaktion auf ein auslösendes Ereignis zu bewirken,
wobei
- die Eingabeeinheit (20) dazu ausgestaltet ist, eine weitere Benutzereingabe zu erfassen, wobei die weitere Benutzereingabe die Information umfasst, dass eine Prüfung eines Gasmessgeräts (1.1, 1.2, 1.3) gewünscht ist, und das auslösende Ereignis die Erfassung der weiteren Benutzereingabe ist, und / oder
- das auslösende Ereignis das Ereignis ist, dass seit dem letzten Vorgang, dass ein eingesetztes Gasmessgerät die Zielgas-Konzentration misst, eine vorgegebene obere Zeitdauer-Schranke verstrichen ist.

10. System umfassend
- einen Ausgabeautomaten (100) nach einem der vorhergehenden Ansprüche,
- ein Gasmessgerät (1.1, 1.2, 1.3),
- eine externe Fluidführungseinheit (41) und
- einen Behälter (40) mit einem Prüfgas,
wobei das Prüfgas ein Zielgas mit einer vorgegebenen Zielgas-Konzentration aufweist,
wobei die externe Fluidführungseinheit (41) eine Fluidverbindung zwischen dem Behälter (40) und dem Gaseinlass (31, 32) herstellt oder herzustellen vermag,
wobei das Gasmessgerät (1.1, 1.2, 1.3) in die Aufnahmeeinheit (10.1, 10.2, 10.3) eingesetzt oder einsetzbar ist,
wobei dann, wenn das Gasmessgerät (1.1, 1.2, 1.3) in die Aufnahmeeinheit (10.1, 10.2, 10.3) eingesetzt ist,
- die Fluidführungs-Anordnung (30, 11.1, 11.2, 11.3) eine Fluidverbindung zwischen dem Gaseinlass (31, 32) und dem Gasmessgerät (1.1, 1.2, 1.3) herstellt und
- das System dazu ausgestaltet ist, zu bewirken oder wenigstens zu ermöglichen, dass eine Gasprobe mit dem Prüfgas vom Behälter (40) zum eingesetzten Gasmessgerät (1.1, 1.2, 1.3) fließt, und
wobei die Steuereinheit (16.1, 16.2, 16.3, 54, 65) dazu ausgestaltet ist,
- zu bewirken, dass das eingesetzte Gasmessgerät (1.1, 1.2, 1.3) die Zielgas-Konzentration in der Gasprobe misst,
- die gemessene Zielgas-Konzentration mit der vorgegebenen Zielgas-Konzentration zu vergleichen und
- dann zu entscheiden, dass das Gasmessgerät (1.1, 1.2, 1.3) nicht intakt ist, wenn die gemessene Zielgas-Konzentration von der vorgegebenen Zielgas-Konzentration um mehr als eine vorgegebene Toleranz abweicht.

11. Ausgabe-Verfahren zum Ausgeben eines Gasmessgeräts (1.1, 1.2, 1.3),
wobei das Ausgabe-Verfahren unter Verwendung eines Ausgabeautomaten (100) durchgeführt wird,
wobei der Ausgabeautomat (100)
- eine Aufnahmeeinheit (10.1, 10.2, 10.3), welche dazu ausgestaltet ist, ein Gasmessgerät (1.1, 1.2, 1.3) aufzunehmen,
- einen Verschluss (13.1, 13.2, 13.3),
- eine Arretiereinheit (14.1, 14.2, 14.3),
- einen Gaseinlass (31, 32),
- eine Fluidführungs-Anordnung (30, 11.1, 11.2, 11.3),
- eine Eingabeeinheit (20) und
- eine signalverarbeitende Steuereinheit (16.1, 16.2, 16.3, 54, 65)
umfasst,
wobei die Fluidführungs-Anordnung (30, 11.1, 11.2, 11.3) den Gaseinlass (31, 32) mit der Aufnahmeeinheit (10.1, 10.2, 10.3) dergestalt verbindet,
dass dann, wenn ein Gasmessgerät (1.1, 1.2, 1.3) in die Aufnahmeeinheit (10.1, 10.2, 10.3) eingesetzt ist, die Fluidführungs-Anordnung (30, 11.1, 11.2, 11.3) eine Fluidverbindung zwischen dem Gaseinlass (31, 32) und dem eingesetzten Gasmessgerät (1.1, 1.2, 1.3) herstellt,
wobei vor der Durchführung des Ausgabe-Verfahrens
- der Verschluss (13.1, 13.2, 13.3) relativ zur Aufnahmeeinheit (10.1, 10.2, 10.3) in einer verschließenden Position ist und verhindert, dass sich das Gasmessgerät (1.1, 1.2, 1.3) aus der Aufnahmeeinheit (10.1, 10.2, 10.3) entnehmen lässt, und
- die Arretiereinheit (14.1, 14.2, 14.3) in einem arretierenden Zustand ist und verhindert, dass der Verschluss (13.1, 13.2, 13.3) in eine geöffnete Position bewegt wird,
wobei das Ausgabe-Verfahren den Schritt umfasst, dass die Eingabeeinheit (20) eine Benutzereingabe erfasst, welche die Information umfasst, dass die Ausgabe eines Gasmessgeräts (1.1, 1.2, 1.3) gewünscht ist,
wobei der Schritt, dass die Benutzereingabe erfasst worden ist, den Schritt auslöst,
dass die Steuereinheit (16.1, 16.2, 16.3, 54, 65) prüft, ob in die Aufnahmeeinheit (10.1, 10.2, 10.3) ein verwendbares Gasmessgerät (1.1, 1.2, 1.3) eingesetzt ist,
wobei ein Gasmessgerät (1.1, 1.2, 1.3) nur dann verwendbar ist, wenn es intakt ist, und wobei es intakt ist, wenn es eine Zielgas-Konzentration bis auf eine vorgegebene Toleranz korrekt zu messen vermag,
wobei dann, wenn festgestellt worden ist, dass in die Aufnahmeeinheit (10.1, 10.2, 10.3) ein verwendbares Gasmessgerät (1.1, 1.2, 1.3) eingesetzt ist, der weitere Schritt durchgeführt wird,
dass die Steuereinheit (16.1, 16.2, 16.3, 54, 65) bewirkt, dass die Arretiereinheit (14.1, 14.2, 14.3) in einen freigebenden Zustand überführt wird,
wobei nach der Überführung in den freigebenden Zustand bewirkt oder wenigstens ermöglicht wird,
dass der Verschluss (13.1, 13.2, 13.3) in die geöffnete Position bewegt wird.

12. Ausgabe-Verfahren nach Anspruch 11,
**dadurch gekennzeichnet, dass**
vor der Durchführung des Ausgabe-Verfahrens die Schritte durchgeführt werden, dass
- ein Gasmessgerät (1.1, 1.2, 1.3) in die Aufnahmeeinheit (10.1, 10.2, 10.3) eingesetzt wird oder eingesetzt ist,
- mittels einer externen Fluidführungseinheit (41) eine Fluidverbindung zwischen einem Behälter (40) mit einem Prüfgas und dem Gaseinlass (31, 32) hergestellt wird,
- wobei das Prüfgas ein Zielgas mit einer vorgegebenen Zielgas-Konzentration aufweist,
- bewirkt oder wenigstens ermöglicht wird, dass eine Gasprobe mit dem Prüfgas durch die externe Fluidführungseinheit (41) und durch die Fluidführungs-Anordnung (30, 11.1, 11.2, 11.3) hindurch zum eingesetzten Gasmessgerät (1.1, 1.2, 1.3) fließt,
wobei anschließend die weiteren Schritte bewirkt werden, dass
- das eingesetzte Gasmessgerät (1.1, 1.2, 1.3) die Zielgas-Konzentration in der Gasprobe misst und
- die Steuereinheit (16.1, 16.2, 16.3, 54, 65) prüft, ob das Gasmessgerät (1.1, 1.2, 1.3) intakt ist oder nicht,
wobei die Steuereinheit (16.1, 16.2, 16.3, 54, 65) das Gasmessgerät (1.1, 1.2, 1.3) als intakt bewertet, wenn die gemessene Zielgas-Konzentration von der vorgegebenen Zielgas-Konzentration um nicht mehr als die vorgegebene Toleranz abweicht.

13. Rückgabe-Verfahren zum Rückgeben eines Gasmessgeräts (1.1, 1.2, 1.3) in
einen Ausgabeautomaten (100),
wobei der Ausgabeautomat (100)
- eine Aufnahmeeinheit (10.1, 10.2, 10.3), welche dazu ausgestaltet ist, ein Gasmessgerät (1.1, 1.2, 1.3) aufzunehmen,
- einen Verschluss (13.1, 13.2, 13.3),
- eine Arretiereinheit (14.1, 14.2, 14.3),
- ein Lesegerät (60, 66) und
- eine signalverarbeitende Steuereinheit (16.1, 16.2, 16.3, 54, 65)
umfasst,
wobei vor der Durchführung des Rückgabe-Verfahrens
- in die Aufnahmeeinheit (10.1, 10.2, 10.3) kein Gasmessgerät eingesetzt ist,
- der Verschluss (13.1, 13.2, 13.3) relativ zur Aufnahmeeinheit (10.1, 10.2, 10.3) in einer verschließenden Position ist und verhindert, dass sich ein Gasmessgerät in die Aufnahmeeinheit (10.1, 10.2, 10.3) einsetzen lässt, und
- die Arretiereinheit (14.1, 14.2, 14.3) in einem arretierenden Zustand ist und verhindert, dass der Verschluss (13.1, 13.2, 13.3) in eine geöffnete Position bewegt wird,
wobei das Rückgabe-Verfahren den Schritt umfasst, dass das Lesegerät (60, 66) eine maschinenlesbare Kennung des Gasmessgeräts (1.1, 1.2, 1.3), das zurückgegeben werden soll, erfasst,
wobei der Schritt, dass die Kennung erfasst worden ist, den Schritt auslöst, dass
die Steuereinheit (16.1, 16.2, 16.3, 54, 65) prüft, ob die Aufnahmeeinheit (10.1, 10.2, 10.3) das Gasmessgerät (1.1, 1.2, 1.3), dessen Kennung erfasst worden ist, aufzunehmen vermag oder nicht,
wobei dann, wenn festgestellt worden ist, dass die Aufnahmeeinheit (10.1, 10.2, 10.3) das Gasmessgerät (1.1, 1.2, 1.3) aufzunehmen vermag,
der weitere Schritt durchgeführt wird, dass die Steuereinheit (16.1, 16.2, 16.3, 54, 65) bewirkt, dass die Arretiereinheit (14.1, 14.2, 14.3) in einen freigebenden Zustand überführt wird, und
bewirkt oder wenigstens ermöglicht wird, dass der Verschluss (13.1, 13.2, 13.3) in die geöffnete Position bewegt wird.

14. Rückgabe-Verfahren nach Anspruch 13,
**dadurch gekennzeichnet, dass**
der Ausgabeautomat (100) zusätzlich
- einen Gaseinlass (31, 32) und
- eine Fluidführungs-Anordnung (30, 11.1, 11.2, 11.3)
umfasst,
wobei die Fluidführungs-Anordnung (30, 11.1, 11.2, 11.3) eine Fluidverbindung zwischen dem Gaseinlass (31, 32) und der Aufnahmeeinheit (10.1, 10.2, 10.3) herstellt und
wobei der Schritt, dass das Gasmessgerät (1.1, 1.2, 1.3) in die Aufnahmeeinheit (10.1, 10.2, 10.3) eingesetzt worden ist, die Schritte auslöst, dass
- mittels einer externen Fluidführungseinheit (41) eine Fluidverbindung zwischen einem Behälter (40) mit einem Prüfgas und dem Gaseinlass (31, 32) hergestellt wird oder hergestellt ist,
wobei das Prüfgas ein Zielgas mit einer vorgegebenen Zielgas-Konzentration aufweist,
- bewirkt oder wenigstens ermöglicht wird, dass eine Gasprobe mit dem Prüfgas durch die externe Fluidführungseinheit (41) und durch die Fluidführungs-Anordnung (30, 11.1, 11.2, 11.3) hindurch zum eingesetzten Gasmessgerät (1.1, 1.2, 1.3) fließt,
- das eingesetzte Gasmessgerät (1.1, 1.2, 1.3) die Zielgas-Konzentration in der Gasprobe misst und
- die Steuereinheit (16.1, 16.2, 16.3, 54, 65) prüft, ob das eingesetzte Gasmessgerät (1.1, 1.2, 1.3) intakt ist oder nicht,
wobei die Steuereinheit (16.1, 16.2, 16.3, 54, 65) das Gasmessgerät (1.1, 1.2, 1.3) als intakt bewertet, wenn die gemessene Zielgas-Konzentration von der vorgegebenen Zielgas-Konzentration um nicht mehr als eine vorgegebene Toleranz abweicht.

15. Rückgabe-Verfahren nach Anspruch 13 oder Anspruch 14,
**dadurch gekennzeichnet, dass**
der Ausgabeautomat (100) eine Kamera (66) und eine signalverarbeitende Bildauswerteeinheit (65) umfasst,
wobei bevorzugt die Kamera (66) dergestalt positioniert ist, dass das Bild das Gasmessgerät (1.1, 1.2, 1.3) zeigt, während das Gasmessgerät (1.1, 1.2, 1.3) sich außerhalb des Ausgabeautomaten (100) befindet, und
wobei der Schritt, dass die Kennung des zurückzugebenden Gasmessgeräts (1.1, 1.2, 1.3) erfasst worden ist, zusätzlich den Schritt auslöst, dass
die Kamera ein Bild des Gasmessgeräts (1.1, 1.2, 1.3) erzeugt und
die Bildauswerteeinheit (65) durch Auswertung des erzeugten Bildes automatisch entscheidet, ob das Gasmessgerät (1.1, 1.2, 1.3) eine optisch erkennbare, insbesondere eine mechanische Beschädigung aufweist oder nicht.
